# EUROPEAN PATENT APPLICATION

(11) **EP 1 541 660 A1**
(43) Date of publication of application: **15.06.2005**
(21) Application number: 03791307.6
(22) Date of filing: 26.08.2003
(51) Int. Cl.: C09K 15/06, A61K 47/26, A61K 47/36, A61K 7/00, C08F 2/40, A23L 1/03, A23L 3/3562, C11B 5/00

(54) **RADICAL REACTION INHIBITORS, METHOD FOR INHIBITION OF RADICAL REACTIONS, AND USE THEREOF**

(30) Priority: 30.08.2002 JP 2002256069
(71) Applicant: Kabushiki Kaisha Hayashibara Seibutsu Kagaku Kenkyujo, Okayama-shi, Okayama 700-0907 (JP)
(72) Inventor: OKU, Kazuyuki, K. K. Hayashibara Seibutsu Kag. Ke., Okayama-shi, Okayama 700-0907 (JP); KUBOTA, Michio, K. K. Hayashibara Seibutsu Kag.Ke., Okayama-shi, Okayama 700-0907 (JP); FUKUDA, Shigeharu, K. K. HayashibaraSeib. Kag.Ke., Okayama-shi, Okayama 700-0907 (JP); MIYAKE, Toshio, K. K. Hayashibara Seib. Kag. Ke., Okayama-shi, Okayama 700-0907 (JP)
(74) Representative: Daniels, Jeffrey Nicholas
(86) International application number: PCT/JP2003/010794
(87) International publication number: WO 2004/020552

(57) **Abstract**

The present invention has an object to provide a radical reaction inhibitory agent for inhibiting unsaturated compounds from decomposing through radical reactions, a method for inhibiting the formation of free radicals from unsaturated compounds and radical reactions thereof, and a composition which is suppressed in radical formation, radical reaction, or progress thereof. The object is solved by establishing a radical reaction inhibitory agent containing as an effective ingredient cyclotetrasaccharide or a mixture of cyclotetrasaccharide and its saccharide derivative(s), a method for inhibiting the formation of free radicals from unsaturated compounds and radical reactions thereof, and a composition which contains the agent and which is suppressed in radical formation, radical reaction, or progress thereof.

## Description

### Technical Field

The present invention relates to radical reaction inhibitory agents and use thereof, more particularly, to novel radical reaction inhibitory agents containing, as an effective ingredient, a non-reducing saccharide composed of four glucose residues linked together for cyclization via the α-1,3 linkage and the α-1,6 linkage, i.e., cyclo{→6}-α-D-glucopyranosyl -(1→3) -α-D-glucopyranosyl - (1→6)-α-D-glucopyranosyl-(1→3)-α-D-glucopyranosyl-(1→} (abbreviated as "cyclotetrasaccharide" hereinafter) or mixtures of cyclotetrasaccharide and its saccharide derivative(s), compositions containing the radical reaction inhibitory agents and unsaturated compounds, and methods for inhibiting radical reaction comprising a step of incorporating the radical reaction inhibitory agents into compositions with unsaturated compounds.

### Background Art

There have been well known that products mainly composed of organic compounds such as lipids, dyes, and synthetic high molecules will be deteriorated in quality and function during their storage as a result of undesired odor occurrence, color changing, color deterioration, hardening, decomposition, quality changing, etc. The following fact has been also known: In food products and pharmaceuticals, peroxides, which are formed through radical reaction, will deteriorate useful ingredients contained therein such as proteins, peptides and/or amino acids, and also augment the reduction of their quality and function. Such peroxides have been focused on their correlation with diseases including life-style related diseases due to their cytotoxic action on cells and tissues.

A major causative reaction, which induces the quality deterioration and the functional reduction and even may form components recognized as affecting human health, is a chemical reaction induced by organic compounds in the absence of catalysts. It can be said that the more organic compounds are susceptible to radicalization the more they are susceptible to the aforesaid quality deterioration, i.e., chemical changing. It can be also said that feasibility of radicalization closely relates to the presence or the absence of intermolecular unsaturated bond and further to their existing status. When once free radicals are formed from organic compounds depending on any causatives, a series of chain reactions among the formed free radicals and other intact molecules or molecular oxygen will progress and result in quality deterioration and functional reduction by a large margin. Since the radicalization of organic compounds is easily induced under ordinary-occurring environmental conditions such as light irradiation and heating, the chemical changing in products induced by a series of chain reactions progressed by free radicals (called "radical reaction", hereinafter) would be a problem inducible in broader fields of food products, cosmetics, pharmaceuticals, chemical industries, etc. Because of this, protection or inhibition of radical reaction would be one of the most important objects to maintain the quality and function of the above products.

As a method for solving the above object, a means most commonly used now in the above-identified fields is to add substances, which are capable of protecting or inhibiting the reactivity of the already-existing free radicals, to the desired products or materials thereof. The inhibitory effect of radical scavengers, however, has a defect that the effect is not durable endlessly.

Under these circumstances, Japanese Patent Kokai No. 2001-123,194 (called "Patent Literature 1", hereinafter) proposes a method for inhibiting the production of volatile aldehydes from fatty acids by incorporating α,α-trehalose and/or maltitol thereunto. To meet recent various dietary habits, there has been desired in development of food materials which would not lower taste, flavor, biting property, etc. , of food products even when digested habitually, but have safe radical reaction inhibitory effect and satisfactory function.

While, in International Application No. WO 01/90,338 (called "Patent Literature 2", hereinafter) or International ApplicationNo. WO 02/10361 (called "Patent Literature 3", hereinafter), the present inventors have disclosed that cyclotetrasaccharide has both deterioration inhibitory effect on lipids and deterioration inhibitory effect on proteins, however, the reason has not yet been clarified. Also, these Patent Literatures 1 to 3 never disclose the fact that cyclotetrasaccharide or a mixture of cyclotetrasaccharide and its saccharide derivative(s) inhibits the radicalization of unsaturated compounds or its successive progress of a series of radical reactions.

### Disclosure of Invention

The first object of the present invention is to provide radical formation inhibitory agents for inhibiting or protecting both the formation of free radicals and the progress of radical reaction, including any reactions where peroxides formed as a result of radical reaction will modify or denature ingredients such as coexisting proteins,peptides,amino acids,etc.,other than unsaturated compounds in order to improve the occurrence of odor, browning, discoloration, hardening, decomposition, and denaturation which are inducible as a result of radical reaction of the unsaturated compounds and the storage of compositions with the decomposed unsaturated compounds; and to improve additional reduction of quality and function of the final products, which are inducible by peroxides, formed as a result of radical reaction, that modify the coexisting ingredients such as proteins, peptides, amino acids, etc., other than the unsaturated compounds. The second object of the present invention is to provide a method for inhibiting the formation of free radicals from unsaturated compounds and their radical reactions using the above radical reaction inhibitory agent. The third object of the present invention is to provide compositions which contains the above radical reaction inhibitory agent and in which radical reaction is inhibited.

To solve the above-identified objects, the present inventors have researched the methods for inhibiting the formation and the inhibition of free radicals and the denaturation of proteins by using saccharides for a relatively long time. As a result, they newly found that cyclotetrasaccharide or a mixture of cyclotetrasaccharide and its saccharide derivative(s) strongly inhibits both the radical formation from unsaturated fatty acids and the progress of radical reaction, and that peroxides formed by radical reaction will modify or denature ingredients such as proteins other than unsaturated substances, and thus they established a radical reaction inhibitory agent, method for inhibiting radical formation, and composition containing the radical reaction inhibitory agent. The present inventors accomplished the present invention by revealing the fact that cyclotetrasaccharide or a mixture of cyclotetrasaccharide and its saccharide derivative(s) inhibits the radical formation and the progress of radical reaction; establishing a radial reaction inhibitory agent containing, as an effective ingredient, cyclotetrasaccharide and its saccharide derivative(s); establishing a method for inhibiting the formation of free radicals and the progress of radical reaction by incorporating the above radical inhibitory agent into a reaction system containing an organic unsaturated compound(s) before or after the formation of free radicals and the induction of radical reaction; and establishing compositions prepared by incorporating the radical reaction inhibitory agent into food products, cosmetics, pharmaceuticals, products of chemical industries etc.

### Best Mode for Carrying Out the Invention

The cyclotetrasaccharide and the mixture of cyclotetrasaccharide and its saccharide derivative(s) usable in the present invention include any of those which are prepared independently of their origins and preparations; fermentation methods, enzymatic methods, organic chemical synthetic methods, etc. Any reaction mixtures obtainable thereby can be arbitrarily used intact as a solution containing cyclotetrasaccharide or a mixture of cyclotetrasaccharide and its saccharide derivative(s) or used after partially or highly purified. The aforesaid cyclotetrasaccharide or the mixture can be prepared, for example, by enzymatic methods using as materials amylaceous substances or saccharides obtainable therefrom; the method for converting panose into cyclotetrasaccharide using α-isomaltosyl-transferring enzyme as disclosed in Patent Literature 2, and the method for producing cyclotetrasaccharide or a mixture of cyclotetrasaccharide and its saccharide derivative(s) obtainable directly from starch using α-isomaltosylglucosaccharide-forming enzyme and α-isomaltosyl-transferring enzyme in combination. These methods can be advantageously feasible because they enable to produce cyclotetrasaccharide and its saccharide derivatives from abundant and low cost amylaceous substances in a relatively higher yield and at a relatively lesser cost. In the present invention, any cyclotetrasaccharides in the form of an anhydrous amorphous, anhydrous, monohydrous, or pentahydrous crystal can be used. Among these cyclotetrasaccharides, those in the form of an anhydrous, monohydrous or anhydrous amorphous crystal have an advantageous dehydrating ability, and therefore, when used in pulverizing and solidifying hydrous matters containing unsaturated compounds, they function as a dehydrating agent and can be advantageously used in preparing high quality powers and solid formulations containing cyclotetrasaccharide as an effective ingredient by adding them to the above hydrous matters.

The term "a mixture of cyclotetrasaccharide and its saccharide derivative(s)" as referred to as in the present invention means either a mixture of cyclotetrasaccharide and its saccharide derivative(s) which one or more glycosyl residues are bound to cyclotetrasaccharide, or a mixture of the above-identified cyclotetrasaccharide and its saccharide derivative(s) along with other saccharide(s). The mixture of cyclotetrasaccharide and its saccharide derivative (s) may include, for example, saccharide mixtures containing cyclotetrasaccharide and its saccharide derivative(s), which one or more glucose residues are bound to one or more of the hydroxyl residues of cyclotetrasaccharide, and/or the saccharide derivative(s) and glucose, maltooligosaccharide, maltodextrin, etc. ; and partially or highly purified products of the above saccharide mixtures prepared by using ion-exchange resins. In addition, any of the following saccharide compositions can be used in the present invention as long as they contain cyclotetrasaccharide; saccharide derivatives obtainable by allowing one or more enzymes having a saccharide-transferring activity such as cyclomaltodextrin glucanotransferase, β-galactosidase, α-galactosidase, and lysozyme to act on a mixture of cyclotetrasaccharide and its saccharide derivative(s), where one or more glucose residues are bound to one or more of the hydroxyl residues of cyclotetrasaccharide, in the presence of monosaccharides, oligosaccharides, and/or polysaccharides as substrates for the enzymes to transfer one or more glycosyl residues such as α-D-glucopyranosyl residue, β-D-galactopyranosyl residue, and β-D-chitosaminyl residue to any one or more of the hydroxyl residues of cyclotetrasaccharide and its saccharide derivative(s); and partially or highly purified ones of the saccharide derivatives.

The term "unsaturated fatty acids" as referred to as in the present invention means hydrocarbons having an unsaturated bond between two carbon atoms (called simply "unsaturated bond", hereinafter), i.e., double or triple bond; and derivatives of the above hydrocarbons, where hydrogen atoms are replaced with other atoms or residues, for example, compounds including lipid acids, alcohols, simple lipids, conjugated lipids, terpenes, synthetic high molecules, and vinyls.

The term "fatty acids" as referred to as in the present invention means chain compounds having one or more carboxyl groups and optionally a branched structure, cyclic structure and/or hydroxyl group, and salts thereof. Examples of such fatty acids include monoene-type fatty acids having only one double bond such as oleic acid, palmitoleic acid, nervonic acid, tsuzuic acid, obtusilic acid, vaccenic acid; polyene-type fatty acids having two or more double bonds, such as linoleic acid, linolenic acid, arachidonic acid, eicosapentaenoic acid, docosapentaenoic acid, docosahexaenoic acid, prostaglandin, thromboxane, and leukotriene; acetylene-type fatty acids having at least one triple bond, such as rylic acid, xymenic acid, erythrogenic acid, crepenic acid, and mycomycin; and polyene-dicarboxylic acid having two or more double bonds and two carboxyl residues, such as muconic acid.

The term "alcohols" as referred to as in the present invention means compounds where hydrogen atoms in chain hydrocarbons are replaced with hydroxyl groups, and includes both monoalcohols having a hydroxyl group and polyalcohols having at least two hydroxy groups. Concrete examples of such include oleyl alcohol.

The term "simple lipids" as referred to as in the present invention means organic compounds which are composed of carbon, hydrogen, and oxygen atoms and which have a hydrocarbon chain corresponding to fatty acid compounds intramolecularly. Representatives of such include dehydration-condensation compounds, i.e., esters, of fatty acid compounds and alcohol compounds or the like. Examples of such are decyl oleate and octyldodecyl oleate which have monoalcohols as their alcohol parts; dioleic acid propylene glycols having propylene glycol as their alcohol parts; monoacyl glycerols, diacyl glycerols, and triacyl glycerols (may be called "neutral fats") having glycerols as their alcohol parts and the above-identified unsaturated fatty acids as their fatty acid parts, as well as having one, two or three fatty acid parts within their single molecule; poly glycerin fatty acid esters having unsaturated fatty acids as their fatty acid parts; and sucrose fatty acid esters, sucrose monooleate, sucrose monolinoleate, and sucrose dioleate, which have sucrose as their alcohol parts. In general, so called "fats and oils" mean compositions containing oil soluble substances mainly composed of triacylglycerol, and they are classified into "fatty oils" and "fats" which exist in a liquid form or a solid form at normal temperature, respectively. Since these fats and oils contain unsaturated fatty acids in general, they will be applicable in practicing the present invention.

The term "conjugated lipids" as referred to as in the present invention means organic compounds which intramolecularly contain hydrocarbon chains corresponding to fatty acid compounds similarly as the above-identified simple lipids, and also contain carbon, hydrogen, oxygen, phosphorus, and nitrogen atoms as constitutive atoms. In general, such conjugated lipids are generally, roughly classified into four groups of glycerophospholipids, glyceroglycolipids, sphingolipids, and sphingoglycolipids. In the present invention, derivatives and partial hydrolyzates of the conjugated lipids such as ceramides thereof are included in the conjugated lipids: Examples of glycerophospholipids are lecithin (phosphatidylcholine), phosphatidylethanolamine, and phosphatidylinositol; examples of glyceroglycolipids are diacylglycerols which have one or more sugar residues such as glucosyl galactosyl residues; examples of sphingolipids are sphingomyelins; and examples of sphingoglycolipids are cerebrosides and ceramides.

The term "terpenes" as referred to as in the present invention means organic compounds, represented by the chemical formula CH₂=C(CH₃)CH=CH₂ composed of a constitutive unit of isoprene, in the form of a chain or cyclic structure. In the present invention, conjugated terpenes which partially have an isoprene unit are included in the above-identified terpenes. Examples of such terpenes are monoterpene, diterpene, triterpene, aqualene, tetraterpene, and carotenoid; and conjugated terpenes such as α-carotene, β-carotene, astaxanthin, canthaxanthin, abscisic acid, vitamin A, and vitamin E.

The term "synthetic high molecules" as referred to as in the present invention means chemically synthesized high molecular substances, which are roughly classified into synthetic rubbers, thermosetting resins, and thermoplastic resins. Since most of the synthetic rubbers have unsaturated bonds, they are surely included within the scope of the present invention. Concrete examples of such are isoprene rubbers, butadiene rubbers, styrene-butadiene rubbers, nitrile rubbers, and nitrile-isoprene rubbers. Examples of the above thermosetting resins are unsaturated polyester resins.

The term "vinyls" as referred to as in the present invention means organic compounds which have a vinyl group represented by the chemical formula CH₂=CH- , a vinylidene group represented by the chemical formula CH₂=C=, or a vinylene group represented by the chemical formula -CH=CH-. Examples of such are olefinic hydrocarbons such as ethylene, propylene, butylene, and isobutylene; polyene hydrocarbons such as butadiene and isoprene; acid vinyl esters such as vinyl acetate and vinyl laurate; acrylate such as methyl acrylate and ethyl acrylate; methacrylate such as methyl methacrylate and ethyl methacrylate; vinyl ethers such as lauryl vinyl ether; vinyl chloride; vinylidene chloride; styrene; acrylonitrile; acryl amide; maleic acid; vitamin D; vitamin K; styryl dyes disclosed in Japanese Patent No. 3,232,512 (Japanese Patent KokaiNo. 343,211/99) applied for by the same applicant as the present invention; indolenine pentamethine cyanine dyes disclosed in International Patent Publication No. WO 01/040,382 (International Patent Application No. PCT/JP00/08298) applied for by the same applicant as the present invention; trimethine cyanine dyes disclosed in Japanese Patent Kokai No. 2002-212,454 (Japanese Patent Application No. 2000-41,001) applied for by the same applicant as the present invention; trimethine cyanine dyes disclosed in International Patent Publication No. WO 01/062,853 (International Patent Application No. PCT/JPOO/09,257) applied for by the same applicant as the present invention; dimethine styryl dyes disclosed in International Patent Publication No. WO 01/019,923 (International Patent Application No. PCT/JP00/06,312) applied for by the same applicant as the present invention; styryl dyes disclosed in Japanese Patent Kokai No. 2001-32,179 (Japanese Patent Application No. 2000-203,873) applied for by the same applicant as the present invention; unsymmetrical indolenine pentamethine cyanine dyes disclosed in Japanese Patent Kokai No. 2001-323,179 (Japanese Patent Application No. 2000-275,764) applied for by the same applicant as the present invention; unsymmetrical trimethine cyanine dyes disclosed in International Patent Publication No. WO 00/061,687 (International Patent Application No. PCT/JP00/02,349) applied for by the same applicant as the present invention; KANKOSO-101 or "PLATONIN™", KANKOSO-201 or PIONIN™, KANKOSO-301 or TAKANAL™, and KANKOSO-401 or "LUMINEX™", which are all listed in "The Japanese Standards of Cosmetic Ingredients, Second Edition, Supplement I", edited by Society of Japanese Pharmacopoeia, published by Yakuji Nippo, Ltd., Tokyo, Japan, (1984).

"Radicalization" as referred to as in the present invention should not be restricted to be one caused by a specific causative. For example, it has been known that unsaturated compounds are radicalized in general by light irradiation and heating in the absence of any catalysts, the action of appropriate catalysts such as metal catalysts, or by the action of other radicals such as active oxygen.

The radical reaction inhibitory agent of the present invention will be useful in a variety of fields, where unsaturated compounds or compositions containing the unsaturated compounds are used as materials, additives, or final products, and where the chemical change of unsaturated fatty acids should be avoided; food products, agriculture/forestry/fisheries, cosmetics, pharmaceuticals, daily goods, chemical industries,dyes,paints,building materials,flavors, chemicals, synthetic fibers, pigments, photosensitive dyes, and optical recording media, as well as the fields of producing materials and additives used in the above fields.

When used in the field of food products where compositions, which contain proteins and lipids as unsaturated fatty acids, are predominant, the radical inhibitory agent of the present invention can be advantageously used as a protein denaturation inhibitory agent to satisfactory maintain the taste and flavor, i.e. , product quality, of the food products because the agent inhibits the radicalization of lipids, the progress of the radical reaction, and the process of both modification and deterioration of proteins induced by lipid peroxides formed by radical reaction. The radical reaction inhibitory agent of the present invention can be advantageously used as a material for health foods because it effectively inhibits peroxides which modify and denature tissues and enzymes present in the tissue even when food products with peroxides are digested. Since cyclotetrasaccharide or a mixture of cyclotetrasaccharide and its saccharide derivative(s) functions as a dietary fiber and has an inhibitory action on the accumulation of body fats, the agent of the present invention can be arbitrarily used as a material for health foods.

In addition, the radical reaction inhibitory agent of the present invention can be arbitrarily applied as a stabilizer to physiologically active substances susceptible to easily lose their activity, as well as to health foods and pharmaceuticals which contain such as physiologically active substances. For example, the agent can be arbitrarily used in preparing high quality health foods, pharmaceuticals, and reagents because it inhibits the denaturation of and stably retains the physiological activities of solutions containing lymphokines such as interferon-α (IFN-α), interferon-β (IFN-β), interferon-γ (IFN-γ), tumor necrosis factor-α (TNF-α), tumor necrosis factor-β (TNF-β), macrophage migration inhibitory factor, colony stimulating factor, transfer factor, and interleukins; solutions containing hormones such as insulin, growth hormone, prolactin, erythropoietin, follicle stimulating hormone, and placental hormone; solutions containing biological preparations such as BCG vaccine, vaccine of Japanese encephalitis virus, vaccine of measles, polio-live-vaccine, smallpox, tetanus toxoid, Antivenenum *Trimeresurus flavoviridis,* human immunoglobulins; chromoproteins such as phycocyanin and phycoerythrin; enzymes such as lipase, elastase, urokinase, protease, β-amylase, isoamylase, glucanase, lactase, and complement system; and peptides or proteins such as blood components.

Since the radical reaction inhibitory agent inhibits the formation of free radicals in tissues and cells and successive series of radical reactions, it can be advantageously used, as an inhibitor for inflammatory reactions accompanied by the formation of free radicals generated in vivo, to prevent and treat dermatitis including atopy, eczema, hives, insect stings, burn, sunburn, inflammatory diseases via immunoreactions, mouth inflammation, gingival inflammation, conjunctivitis, and inflammatory diseases of organs such as gastritis, colitis, idiopathic ulcerative colitis,and Crohn's disease.

The radical reaction inhibitory agent of the present invention preferably contain cyclotetrasaccharide or a mixture of cyclotetrasaccharide and its saccharide derivative(s) in an amount of at least about 1% (w/w) (the symbol "% (w/w)" is abbreviated as "%" hereinafter, unless specified otherwise) in terms of anhydrous cyclotetrasaccharide, preferably, at least 10%, and more preferably, at least 30%. As long as exerting the desired effect of inhibiting the radical formation and the progress of radical reaction, the radical reaction inhibitory agent of the present invention can be cyclotetrasaccharide per se or a mixture of cyclotetrasaccharide and its saccharide derivative(s), which can optionally contain other saccharide(s) such as glucose, isomaltose, maltose, maltotriose, maltodextrin, etc. In the case that compositions to be incorporated with the radical reaction inhibitory agent may contain substances such as amino acids having amino residues intramolecularly and that such compositions may problematically cause the quality reduction of effective ingredients contained in the compositions and/or the compositions per se, due to the Maillard reaction induced by contaminated reducing saccharide(s) such as glucose, the radical reaction inhibitory agent of the present invention should include, preferably, cyclotetrasaccharide in an amount of at least 98%, more preferably, at least 99%, and most preferably, 99.5%. Further, those, which are prepared by hydrogenating cyclotetrasaccharide or a mixture of cyclotetrasaccharide and its saccharide derivative(s) along with the coexisting reducing saccharides to lower the reducibility, can be also used as the radical reaction inhibitory agent. Since cyclotetrasaccharide is a stable saccharide, it can be arbitrarily used in combination with one or more of the following substances, as long as they do not lower the quality of compositions to be incorporated with the radical reaction inhibitory agent of the present invention; radical scavengers and others such as reducing saccharides, non-reducing saccharides, sugar alcohols, water-soluble polysaccharides, inorganic salts, emulsifiers, antioxidants, and substances with chelate action, which can be used depending on the purpose of improving dispersibility, bulk, or the like. If necessary, the radical reaction inhibitory agent can be used in combination with adequate amounts of conventional colors, flavors, preservatives, stabilizers, etc. The radical reaction inhibitory agent thus obtained can be used independently of its form; a syrup, massecuite, paste, powder, crystal, granule, or tablet.

Examples of the non-reducing saccharides usable in combination with the radical reaction inhibitory agent of the present invention are trehalose, i.e., one or more of α,α-trehalose, α,β-trehalose, and β,β-trehalose; and sugar alcohols such as maltitol, which are all desirably used because of their satisfactory inhibitory effect on the deterioration of lipids and on the formation of aldehydes from lipids. Among which, α,α-trehalose is particularly preferably used because it has a quite high level of the above effect.

When used in the desired fields in combination with conventional antioxidation methods used in such fields, the radical reaction inhibitory agent of the present invention more effectively inhibits radical reaction than in the case of its sole use. The antioxidation methods applicable to the present invention include, for example, those which use antioxidants, free oxygen absorbers, radical scavengers, or metal chelates; those to interrupt contacting with oxygen by encapsulating in capsules, coating, preserving in shielded vessels, or injecting inactive gases; and those to preserve under light-shielded and low-temperature conditions. One or more of these methods can be used alone or in an appropriate combination.

The radical scavengers usable in the present invention include any compounds used to effectively trap radicals formed during the progress of reactions, and one or more of which can be arbitrarily selected depending on the use of the radical reaction inhibitory agent of the present invention. For example, vitamins such as L-ascorbic acid including derivatives thereof, vitamin B₂ including derivatives thereof, hesperidin including derivatives thereof, and rutin including derivatives thereof; flavonoids such as polyphenols; dibutylhydroxytoluene; galvinoxyl; hydroquinone; and hydroquinone derivatives. From among the above unsaturated compounds, for example, vitamin A, vitamin E and derivatives thereof as representatives of terpene; and unsaturated compounds such as docosapentaenoic acid and docosahexaenoic acid as representatives of unsaturated fatty acids can be arbitrarily employed depending on use.

The terms of "incorporating cyclotetrasaccharide or a mixture of cyclotetrasaccharide and its saccharide derivative(s)" and "incorporating as an effective ingredient the radical reaction inhibitory agent containing the above cyclotetrasaccharide or the mixture" as referred to as in the present invention mean that, depending on use, any one of the above cyclotetrasaccharide, the mixture, and the radical reaction inhibitory agent is allowed to directly contact with other ingredients in any step from among the stage of handling raw materials through the stage of obtaining the final products, or to already processed products by using conventional methods, for example, mixing, kneading, dissolving, melting, sprinkling, suspending, emulsifying, soaking, permeating, spreading, applying, coating, spraying, injecting, crystallizing, and solidifying. The cyclotetrasaccharide or the mixture of cyclotetrasaccharide and its saccharide derivative(s) usable in the present invention should not be restricted to a specific form; one or more forms of a syrup, massecuite, solid and powder can be arbitrarily selected for use.

The amount of cyclotetrasaccharide or a mixture of cyclotetrasaccharide and its saccharide derivative(s) used in the present invention is not specifically restricted as long as it effectively exerts the desired inhibitory effect on the formation of free radicals from unsaturated compounds and/or on the progress of radical reaction, however, it is preferably incorporated into the unsaturated compounds in an amount of not less than about 0.01% but less than about 99.9%, more preferably, not less than about 1.0% but less than about 90% in terms of anhydrous cyclotetrasaccharide to the compounds. Usually, when used in an amount of less than 0.01%, it is insufficient to inhibit the formation of free radicals from unsaturated fatty acids and the radical reaction of the compounds.

The following experiments explain in detail the inhibition of the formation of free radicals from unsaturated compounds and/or the progress of radical reaction of the compounds by cyclotetrasaccharide or a mixture of cyclotetrasaccharide and its saccharide derivative(s):

### Experiment 1-1: Preparation of cyclotetrasaccharide and saccharide derivatives thereof

In accordance with the method in Example 2 in Patent Literature 3, a syrup containing, on a dry solid basis (d.s.b.), 61.7% of cyclotetrasaccharide and 5.1% of a saccharide derivative of cyclotetrasaccharide. Similarly, in accordance with the method in Example 3 in Patent Literature 3, a cyclotetrasaccharide crystal, pentahydrate, with a purity of 98.5%, d.s.b., was prepared by sequentially subjecting purification, concentration, drying, and crystallization to a mixture of cyclotetrasaccharide and a saccharide derivative thereof was prepared from tapioca starch as a material. Further, in accordance with the method in Experiment 31 or 32 in Patent Literature 3, the above crystal was dried to obtain a cyclotetrasaccharide monohydrate powder and an anhydrous crystalline cyclotetrasaccharide powder.

### Experiment 1-2: Influence of cyclotetrasaccharide on the inhibition of radical formation

An experiment for examining the influence of cyclotetrasaccharide on the inhibition of radical formation was conducted as follows: To 50 µl of a solution containing 1 mM hydrogen peroxide (H₂O₂) were sequentially added 50 µl of a solution containing 89 mM 5,5-dimethyl-1-pyrroline-oxide (DMPO), commercialized by Wako Pure Chemical Industries, Ltd., Tokyo, Japan; 50 µl of a solution containing 15 mM, 38 mM, 75 mM, or 150 mM cyclotetrasaccharide prepared using the syrup containing cyclotetrasaccharide and the saccharide derivative thereof, crystalline cyclotetrasaccharide pentahydrate, crystalline cyclotetrasaccharide monohydrate, and anhydrous crystalline cyclotetrasaccharide, respectively, which had been obtained in Experiment 1-1, or 50 µl of distilled water; 50 µl of a solution containing 1 mM iron sulfate and 1 mM diethylenetriamine-N,N,N',N",N"-5-acetate (DTPA) commercialized by Wako Pure Chemical Industries, Ltd., Tokyo, Japan. Each of the resulting mixtures was mixed to initiate reaction, injected into a cell for measuring electron spin resonance spectrum (ESR), set to a detector, and measured for formation level of hydroxy radical at a prescribed period of time after initiating reaction. As a positive control, in place of cyclotetrasaccharide, "TREHA™" , an α,α-trehalose product commercialized by Hayashibara Shoji Co. , Ltd. , Okayama, Japan, was measured similarly as above. For measurement of ESR, "Free Radical Monitor JES-FR30" was used, and the formation level of hydroxy radical was determined by calculating a ratio of a peak level detected first after initiating the measurement among the characteristic signal peaks characteristic of 5,5-dimethyl-1-pyrroline-oxide-OH (hydroxy radical), used as a spin trapping agent, and a peak level of Mn²⁺ of an external standard installed in the apparatus of "JES-FR30", and expressed as relative values in Table 1. In Table 1, only the results for crystalline cyclotetrasaccharide pentahydrate are shown because four characteristic peaks of 5,5-dimethyl-1-pyrroline-oxide-OH (hydroxy radical) were similarly observed in any of the above syrup containing the mixture of cyclotetrasaccharide and its saccharide derivative, crystalline cyclotetrasaccharide pentahydrate, crystalline cyclotetrasaccharide monohydrate, or anhydrous crystalline cyclotetrasaccharide.

**Table 1**

| | Amount of formed hydroxy radical (Hydroxy radical formation (%)) | | | | |
|---|---|---|---|---|---|
| Concentration of saccharide (mM) | 0 | 15 | 38 | 75 | 150 |
| Cyclotetrasaccharide | 2.995 (100) | 1.009 (34) | 0.265 (9) | 0.228 (8) | 0.125 (4) |
| α,α-Trehalose | 2.995 (100) | 1.974 (66) | 0.692 (23) | 0.236 (8) | 0.194 (6) |
| Formation percentage (%) = (A/B) x 100 A: Formation level of hydroxy radical for the system with different saccharide concentrations B: Formation level of hydroxy radical for the system with no saccharide | | | | | |

### Results

The formation level of hydroxy radical lowered as the increase of the amount of cyclotetrasaccharide added, i.e., the level of hydroxy radical in the systems with cyclotetrasaccharide lowered to about eight percent and about four percent at 75 mM and 150 mM cyclotetrasaccharide, respectively, as compared with the system with no saccharide. In the case of α,α-trehalose, it showed substantially the same formation levels of hydroxy radical at the same concentrations as in the above. In the range of relatively low saccharide concentrations of 38 mM or lower, systems with cyclotetrasaccharide gave formation percentages (%) of hydroxy radical of about 34% and about 9% at concentrations of 15 mM and 38 mM cyclotetrasaccharide, respectively, as compared with that for the system with no saccharide, revealing that the formation level of hydroxy radical was strongly inhibited in the systems with cyclotetrasaccharide. While the systems with α,α-trehalose gave about 66% and 23% at concentrations of 15 mM and 38 mM, respectively, i.e., about two-fold higher levels of the formation percentage (%) of hydroxy radical as those for the systems of cyclotetrasaccharide. Based on the results, it was revealed that cyclotetrasaccharide has a clearly higher inhibitory effect on the formation of hydroxy radical than α,α-trehalose.

### Experiment 1-3: Influence of cyclotetrasaccharide on the oxidation inhibition for low density lipoprotein (LDL)

An experiment for examining the influence of cyclotetrasaccharide on the oxidation inhibition for low density lipoprotein (LDL) was conducted as follows: Two and half milliliters of 0.5 mg/ml of a low density lipoprotein (LDL) in phosphate buffered saline (PBS, pH 7.4) was mixed with 2.5 ml of 100 mM PBS solution containing any one of the crystalline cyclotetrasaccharide pentahydrate, crystalline cyclotetrasaccharide monohydrate, and anhydrous crystalline cyclotetrasaccharide, which had been prepared in Experiment 1-1, in PBS or 2. 5 ml of PBS free of cyclotetrasaccharide. The resulting mixture was admixed with 50 µl of 100 mM 2,2'-azo-bis(2-amidinopropane)dihydrochloride (AAPH) in PBS (pH7.4) and reacted at 37°C. The reaction was continued for 240 min to examine changes on time course. Each reaction mixture was sampled at 0, 30, 60, 120, 180, and 240 min after initiating the reaction, and the samples were measured for absorbance at a wavelength of 234 nm that proportionately increased as the increase of formation level of LDL oxide. Table 2 shows the change on time course of the absorbance at a wavelength of 234 nm for LDL oxide detected in this experiment. In Table 2, the results for crystalline cyclotetrasaccharide pentahydrate are only shown because crystalline cyclotetrasaccharide pentahydrate, crystalline cyclotetrasaccharide monohydrate, and anhydrous crystalline cyclotetrasaccharide gave similar results. Also, the influence of cyclotetrasaccharide on the oxidation of a low density lipoprotein (LDL) by AAPH was examined by mixing a 2.5 ml solution containing 0.5 mg/ml of a low density lipoprotein (LDL) in PBS with 2.5 ml of a PBS solution in which the crystalline cyclotetrasaccharide pentahydrate, prepared in Experiment 1-1, had been dissolved to give a concentration of 2 mM, 10 mM or 20 mM, or 2. 5 ml of PBS with no cyclotetrasaccharide; and measuring for absorbance at a wavelength of 234 nm for each of the resulting mixtures similarly as above. Table 3 shows the relationship between the amount of cyclotetrasaccharide added and the level of oxidized LDL, i.e., the absorbance at a wavelength of 234 nm at 120 min after initiating the reaction.

**Table 2**

| Time elapsed after adding AAPH (min) | Absorbance at a wavelength of 234 nm In | | Formation percentage (%) |
|---|---|---|---|
| | In the absence of cyclotetrasaccharide | In the presence of cyclotetrasaccharide (50 mM) | |
| 0 | 0.0 | 0.0 | - |
| 30 | 0.127 | 0.018 | 14 |
| 60 | 0.211 | 0.042 | 20 |
| 120 | 0.215 | 0.046 | 21 |
| 180 | 0.245 | 0.056 | 23 |
| 240 | 0.279 | 0.064 | 23 |
| Formation percentage (%) = (A/B) x 100 A: Absorbance at each time for the system with cyclotetrasaccharide B: Absorbance at each time for the system with no cyclotetrasaccharide | | | |

**Table 3**

| Concentration of cyclotetrasaccharide (mM) | Absorbance at a wavelength of 234 nm | Formation percentage (%) |
|---|---|---|
| | Average | |
| 0 | 0.215 | 100 |
| 1 | 0.163 | 76 |
| 5 | 0.076 | 35 |
| 10 | 0.028 | 13 |
| Formation percentage (%) = (A/B) x 100 A: Absorbance at each time for the system with cyclotetrasaccharide B: Absorbance at each time for the system with no cyclotetrasaccharide | | |

### Results

As shown in Table 2, it was revealed that both of the systems with no cyclotetrasaccharide (0 mM) and with cyclotetrasaccharide (50 mM) gave an increase of the level of LDL oxide in a time dependent manner over 60 min after initiating the reaction, and roughly reached plateau. The system with cyclotetrasaccharide was observed to have a strong inhibitory effect on the formation of LDL oxide, because the formation percentage (%) of LDL oxide at 240 min after initiating the reaction was about 23% as compared with the system with no cyclotetrasaccharide. As evident from the results in Table 3, the formation percentage (%) of LDL oxide lowered as the increase of the amount of cyclotetrasaccharide added, and it was inhibited up to about 13% in the system with 10 mM of cyclotetrasaccharide as compared with the system with no cyclotetrasaccharide. Based on these results, cyclotetrasaccharide effectively inhibits the oxidization of LDL.

### Experiment 1-4: Influence of cyclotetrasaccharide on the radical oxidation of linoleic acid

An experiment for examining the influence of cyclotetrasaccharide on the radical oxidation of linoleic acid was conducted as follows:

### Assay for the influence of cyclotetrasaccharide on the oxidation of linoleic acid

One milliliter of a solution containing 11.7 mg/ml linoleic acid in ethanol, one milliliter containing 50 mM phosphate buffer (pH 7.2), one milliliter of a solution containing 100 mM 2,2-azo-bis(2-amidinopropane)dihydrochloride (AAPH), and two milliliters of a solution containing 200 mM of the crystalline pentahydrate prepared in Experiment 1-1 in 50 mM PBS (pH 7.2) were mixed and allowed to stand at 37°C while being sampled at 0, 1, 2 and 6 hours after the mixing, followed by assaying the formation levels of conjugated diene formed in the test samples and of the reaction product with thiobarbituric acid (TBARS). As a negative control, a system of PBS with no cyclotetrasaccharide was provided and measured similarly as above. Conjugated diene was quantified by measuring the absorbance of test samples at a wavelength of 234 nm according to the method by Okamura *et al*., in *Journal* of *Agricultural and Food* Chemistry, Vol. 42, page 1,612 (1994). TABRS was quantified by allowing the sampled test specimens to react with thiobarbituric acid and measured for absorbance of the reaction mixture at a wavelength of 532 nm according to the method by Ohgawara et al., in *Journal* of *Lipid Research*, Vol. 19, page 1,053 (1978). The quantification of the formed TEARS in test samples was determined with a calibration curve provided in such a manner of diluting 1,1,3,3-tetraethoxypropane with ethanol into 10, 50 and 100 µg/ml dilutes, allowing the dilutes with thiobarbituric acid similarly as the test samples, measuring the absorbance of the resulting mixtures similarly as above, and drawing calibration curve based on the measured absorbances and the amount of TBARS calculated theoretically. The quantification of the formed conjugated diene in the test samples was determined by calculating the absorbances of the samples obtained in this experiment, based on the molar absorbance coefficient of molecules disclosed in *Journal* of *Agricultural and Food Chemistry,* Vol. 42, page 1,612 (1994). Tables 4 and 5 show the results on measurement for the formation level of conjugated diene and TBARS, respectively.

**Table 4**

| | Formed conjugated diene (µg/g-linoleic acid) (Conjugated diene formation: (%) ) | | | |
|---|---|---|---|---|
| Reaction time (hr) | 0 | 1 | 2 | 6 |
| None | 0.27 (100) | 9.43 (100) | 17.98 (100) | 59.21 (100) |
| Cyclotetrasaccharide | 0.27 (100) | 5.16 (55) | 9.15 (51) | 23.83 (40) |
| Formation percentage (%) = (A/B) x 100 A: Formation level of conjugated diene at each time for the system with cyclotetrasaccharide B: Formation level of conjugated diene at each time for the system with no cyclotetrasaccharide | | | | |

**Table 5**

| | Formed TBARS (µg/g-linoleic acid) (TBARS formation:(%)) | | | |
|---|---|---|---|---|
| Reaction time (hr) | 0 | 1 | 2 | 6 |
| None | 0.00 (-) | 2.76 (100) | 10.26 (100) | 26.43 (100) |
| Cyclotetra-saccharide | 0.00 (-) | 1.52 (55) | 3.55 (35) | 7.97 (30) |
| Formation percentage (%) = (A/B) x 100 A: Formation level of TBARS at each time for the system with cyclotetrasaccharide B: Formation level of TBARS at each time for the system with no cyclotetrasaccharide | | | | |

### Result

As evident form the results in Table 4, the formation level of conjugated diene for the systems with cyclotetrasaccharide was inhibited up to about 40% at six hours after initiating the reaction, as compared with the system with no cyclotetrasaccharide. As evident from the results in Table 5, the formation level of TBARS was inhibited up to about 30% at six hours after initiating the reaction, as compared with the system with no cyclotetrasaccharide. These results revealed that cyclotetrasaccharide effective inhibits the formation of conjugated diene and TBARS from linoleic acid through radical reaction.

### Experiment 1-5: Influence of cyclotetrasaccharide and other saccharides on the inhibition of modifying proteins and lysine by lipid peroxides

By using cyclotetrasaccharide, maltitol, α,α-trehalose, sucrose, and mixtures thereof, an experiment for examining the influence of saccharides on the modification and denaturation of proteins and lysine by 2,4-decadienal and malondialdehyde (MDA), which are representatives of lipid peroxides formed from lipids through radical reaction, was conducted as shown below with an index of the formation of OLAARPs, i.e., protein carbonyl and reaction products of oxidized lipids/amino acids, which are aldehyde adducts formed by reacting the above lipid peroxides with proteins or lysine. In this experiment, saccharide solutions were prepared to give an equal total saccharide molar concentration in each solution.

### Preparation of testing saccharide solutions

Saccharide solutions for testing were prepared by dissolving into five milliliters of PBS (50 mM, pH 7.4) 500 mg of the crystalline cyclotetrasaccharide pentahydrate prepared in Experiment 1-1; 250 mg of "MABIT™", an anhydrous crystalline maltitol, commercialized by Hayashibara Shoji Co., Ltd., Okayama, Japan; 250 mg of "TREHA™", a crystalline trehalose hydrate, commercialized by Hayashibara Shoji Co., Ltd., Okayama, Japan; or 250 mg of sucrose in a special reagent grade, commercialized by Pure Chemical Industries, Ltd., Tokyo, Japan. As a test for a combination of two types of saccharides, it was used a solution prepared by dissolving 250 mg of cyclotetrasaccharide and 125 mg of other saccharide in five milliliters of PBS; and as a test for a combination of three types of saccharides, i.e., cyclotetrasaccharide, hydrous crystalline α,α-trehalose, and anhydrous crystalline maltitol, it was used a solution prepared by dissolving 166.7 mg of cyclotetrasaccharide and 83.3 mg of each of the other saccharides in five milliliters of PBS. As a negative control, PBS was used.

### Experimental method

To five milliliters of either of the above saccharide solutions for testing or the negative control was added 10 mg of calf serum albumin (BSA) or L-lysine, followed by dissolving. To each of the resulting solutions was added 25 µg of 2,4-decadienal or malondialdehyde (MDA) as a lipid peroxide, followed by reaction at 37°C for 24 hours. After completion of the reaction, protein carbonyl and reaction products of oxidized lipids/amino acids(OLAARPs) formed as aldehyde adducts were quantified on the calorimetric method. Table 6 shows the result of the reaction of BSA with the lipid peroxide, and Table 7 shows the result of the reaction of L-lysine with the lipid peroxide. The formation levels of protein carbonyl and OLAARPs were determined based on the absorbances obtained in this experiment, in terms of the molar absorbance coefficient for each molecule as disclosed in *Biochim. Biophys. Acta,* No. 1,258, pp. 319-327 (1985).

### Method for quantifying protein carbonyl

After reacting the lipid peroxide with either the negative control or any one of the saccharide solutions for testing into which BSA had been dissolved, two milliliters of any of the resulting mixtures were admixed with two milliliters of 12.5 mM dinitrophenylhydrazine(DNPH)/2.5 N-hydrochloride, and sequentially allowed to stand at ambient temperature for one hour, admixed with two milliliters of 30% trichloroacetic acid (TCA), and allowed to stand at 0°C for one hour, followed by centrifugation at 10,000 rpm for 15 min, washing the resulting precipitate four times with two milliliters of ethanol/ethyl acetate (1:1), respectively, adding two milliliters of 6 M-guanidine-hydrochloride/20 mM-NaPi/trifluoroacetic acid (TFA, pH 2.3) to the resultant mixture, allowing the mixture to stand at 37°C for 30 min, and measuring the absorbance of the resulting mixture at a wavelength of 370 nm, ε=22,000 M⁻¹cm⁻¹. After reacting the lipid peroxide with either the negative control or any one of the saccharide solutions for testing into which L-lysine had been dissolved, two milliliters of any one of the resulting mixtures were admixed with two milliliters of a 12.5 mM-dinitrophenylhydrazine(DNPH)/2.5 N-hydrochloride solution, and measuring the absorbance of the resulting mixture at a wavelength of 370 nm, ε=22,000 M⁻¹cm⁻¹.

### Method for quantifying OLAARPs

After reacting the lipid peroxide with either the negative control or any one of the saccharide solutions for testing into which BSA had been dissolved, one milliliter of any one of the resulting mixtures was admixed with 150 µl of Ehrlich's reagent containing 200 mg of dimethylbenzaldehyde, two milliliters of ethanol, and eight milliliters of 3.5 N-hydrochloric acid, followed by standing at 45°C for 30 min. To each of the resulting mixtures was added 600 µl of 30% trichloroacetic acid, and the mixture was allowed to stand at 0°C for one hour and centrifuged at 10,000 rpm for 15 min to obtain a precipitate, followed by washing the resulting precipitate four times with two milliliters of ethanol/ethyl acetate (1:1), respectively, adding 1.5 ml of 6M-guanidine hydrochloride/20 mM-NaPi/TFA (pH 2.3) to the resultant, allowing the mixture to stand at 37°C for 30 min, and measuring the absorbance of the resulting mixture at a wavelength of 580 nm, ε=35,000 M⁻¹cm⁻¹. After reacting the lipid peroxide with either the negative control or any one of the saccharide solutions for testing into which L-lysine had been dissolved, one milliliter of any one of the resulting mixtures were admixed with two milliliters of diethylether to remove intact lipid peroxide, admixed with Ehrlich's reagent, and allowed to stand at 45°C for 30 min. To any one of the resulting mixtures was added two milliliters of a 12.5 mM DNPH/2.5 N-hydrochloride solution, and the resulting mixture was allowed to stand at 45°C for 30 min, followed by measuring the absorbance of the resulting mixture at a wavelength of 580 nm, ε=35,000 M⁻¹cm⁻¹.

**Table 6**

| Measurement index | | Amount of aldehyde adducts (µmol/g-BSA) | | | |
|---|---|---|---|---|---|
| | | Formed Protein carbonyl (Formation: (%) ) | | Formed OLAARPs (Formation: (%) ) | |
| Added lipid peroxide | | 2,4-DD* | MDA** | 2,4-DD* | MDA** |
| Added saccharide | None | 14.16 (100) | 2.42 (100) | 4.21 (100) | 5.83 (100) |
| | Cyclotetrasaccharide | 6.34 (45) | 1.09 (45) | 2.92 (69) | 4.68 (80) |
| | α,α -Trehalose | 8.26 (58) | 1.45 (60) | 2.67 (63) | 3.24 (56) |
| | Maltitol | 7.51 (53) | 1.29 (53) | 2.83 (67) | 2.78 (48) |
| | Sucrose | 14.47 (102) | 2.23 (92) | 4.77 (113) | 7.75 (133) |
| | Cyclotetrasaccharide α,α -Trehalose | 7.13 (50) | 1.17 (48) | 2.48 (59) | 3.86 (66) |
| | Cyclotetrasaccharide Maltitol | 7.02 (50) | 1.21 (50) | 2.88 (68) | 3.72 (64) |
| | Cyclotetrasaccharide Sucrose | 9.25 (65) | 1.57 (65) | 3.15 (75) | 5.22 (90) |
| | Cyclotetrasaccharide α,α -Trehalose Maltitol | 6.78 (48) | 1.18 (49) | 2.81 (67) | 5.18 (89) |

| | | | | | |
|---|---|---|---|---|---|
| *: 2,4-Decadienal | | | | | |
| **: Malondialdehyde Formation percentage (%) = (A/B) x 100 A: Formation level of aldehyde adducts at each time for the system with cyclotetrasaccharide B: Formation level of aldehyde adducts at each time for the system with no cyclotetrasaccharide | | | | | |

**Table 7**

| Measurement index | | Amount of aldehyde adducts (µmol/g-L-lysine) | | | |
|---|---|---|---|---|---|
| | | Formed Protein carbonyl (Formation: (%) ) | | Formed OLAARPs (Formation: (%) ) | |
| Added lipid peroxide | | 2,4-DD* | MDA** | 2,4-DD* | MDA** |
| Added saccharide | None | 13.46 (100) | 6.00 (100) | 8.12 (100) | 8.64 (100) |
| | Cyclotetrasaccharide | 6.42 (48) | 3.93 (66) | 5.44 (67) | 3.39 (39) |
| | α,α-Trehalose | 7.47 (55) | 3.99 (67) | 5.72 (70) | 3.24 (38) |
| | Maltitol | 6.81 (51) | 3.29 (55) | 5.90 (73) | 7.23 (84) |
| | Sucrose | 10.92 (81) | 5.75 (96) | 7.54 (93) | 8.13 (94) |
| | Cyclotetrasaccharide α,α-Trehalose | 7.13 (53) | 3.17 (53) | 5.48 (67) | 4.86 (56) |
| | Cyclotetrasaccharide Maltitol | 7.02 (50) | 3.21 (54) | 5.88 (72) | 4.72 (55) |
| | Cyclotetrasaccharide Sucrose | 9.25 (65) | 4.57 (76) | 6.45 (79) | 6.22 (72) |
| | Cyclotetrasaccharide α,α-Trehalose Maltitol | 6.78 (50) | 3.18 (53) | 5.81 (72) | 5.83 (67) |

| | | | | | |
|---|---|---|---|---|---|
| *: 2,4-Decadienal | | | | | |
| **: Malondialdehyde Formation percentage (%) = (A/B) x 100 A: Formation level of aldehyde adducts at each time for the system with cyclotetrasaccharide B: Formation level of aldehyde adducts at each time for the system with no cyclotetrasaccharide | | | | | |

### Results

As evident from the results in Table 6, in the test systems with cyclotetrasaccharide, the saccharide inhibited the formation of protein carbonyl from BSA, induced by the addition of 2,4-decadienal or malondialdehyde (MDA) as a lipid peroxide, to a level of about 45% of that of the control system with 2,4-decadienal or malondialdehyde (MDA) but with no cyclotetrasaccharide. Such an inhibitory effect was also found as in the case of the systems with α,α-trehalose or maltitol in place of cyclotetrasaccharide, however, the level of their inhibitory effect was lower than that of cyclotetrasaccharide. In the test systems with cyclotetrasaccharide, the formation level of OLAARs from BSA induced by the addition of 2,4-decadienal or MDA was inhibited to a level of about 69% for 2,4-decadienal or about 80% for MDA, as compared with the control system with no saccharide. The inhibitory effect on the formation of OLAARPs was found even by the addition of α,α-trehalose or maltitol, and the level of which was substantially the same as compared with that of cyclotetrasaccharide in the system with 2,4-decadienal, and it was higher than that of cyclotetrasaccharide in the system with MDA. While, sucrose substantially did not inhibit the formation of the aforesaid aldehyde adducts, but increased the formation of OLAARPs compared with that of the system with no saccharide. In the systems admixed with two or three saccharides, the formation level of protein carbonyl and OLAARPs from BSA was inhibited compared with the system with no saccharide, and the production enhancement of these aldehyde adducts was not found even when sucrose was used in combination.

As evident from the results in Table 7, the addition of cyclotetrasaccharide inhibited the formation of protein carbonyl from L-lysine by the addition of 2,4-decadienal or MDA to a level of about 48% for 2,4-decadienal or about 66% for MDA, as compared with the system with no saccharide. The formation level of OLAARPs from L-lysine was inhibited by the addition of cyclotetrasaccharide to a level of about 67% for decadienal and about 39% for MDA, as compared with the system with no saccharide. The inhibition of formation of protein carbonyl and OLAARPs from L-lysine by the addition of 2,4-decadienal or MDA was also found when α,α-trehalose and maltitol were added in place of cyclotetrasaccharide, and the inhibitory level of which was substantially the same or slightly lower than that of cyclotetrasaccharide except that, among the saccharides tested, maltitol gave the highest level of inhibitory effect on the formation of protein carbonyl by the addition of 2,4-decadienal. Similarly as in the case of the above BSA, sucrose substantially neither gave an inhibitory effect on the formation of protein carbonyl or OLAARPs as aldehyde adducts nor augmented the formation of such aldehyde adducts. In the systems with two or three saccharides, the formation of protein carbonyl and OLAARPs from L-lysine was inhibited, and the combination use of saccharides did not augment the formation of such aldehyde adducts. Based on these results, it was revealed that cyclotetrasaccharide effectively inhibits the modification and the denaturation of BSA and L-lysine induced by lipid peroxides formed from lipids as unsaturated compounds through radical reaction. It was also revealed that cyclotetrasaccharide does not augment the above-identified modification and denaturation by lipid peroxides even when used with other saccharides in combination.

### Experiment 1-6: Influence of cyclotetrasaccharide on small intestinal mucous disorders by radicals

As a model system for examining the influence of cyclotetrasaccharide on small intestinal mucous disorders, there was provided a system for examining the influence of cyclotetrasaccharide on the oxidation of a rat small intestinal mucosal enzyme solution by2,2-azo-bis(2-amidinopropane)-dihydrochloric acid (AAPH).

### Assay for the influence of cyclotetrasaccharide on the oxidation of rat small intestinal mucosa

To two grams of a rat small intestinal mucosal acetone powder, commercialized by Sigma Chemical Company, St. Louis, Missouri, USA, was added 20 ml of 50 mM PBS (pH 7.2) and homogenized in ice bath. The mixture was centrifuged at 10,000 rpm for 10 min to obtain a supernatant for use as a rat small intestinal mucosal enzyme solution containing 10 mg/ml of proteins. A half milliliter of the rat small intestinal mucosal enzyme solution, 0.5 ml of a 100 mM AAPH solution, and 0.5 ml of a 200 mM of the crystalline cyclotetrasaccharide pentahydrate prepared in Experiment 1-1 were mixed, followed by incubation at 37°C for two hours. During the incubation, the mixture was sampled at two and six hours after initiating the incubation for quantifying the formed conjugated diene, while the specimens sampled at six hours after initiating the incubation were quantified for the formed TBARS. Table 8 shows the results for the data on the formation level of conjugated diene in the rat small intestinal mucosal enzyme solution formed by radical oxidation, and Table 9 shows the results for the data on the formation level of TBARS.

### Assay for the influence of cyclotetrasaccharide on the oxidation of rat small intestinal mucosal enzyme

One milliliter of the above rat small intestinal mucosal enzyme solution, onemilliliterof a 100 mM AAPH solution, onemilliliter of a 50 mM phosphate buffer (pH 7.2), and one milliliter of a 400 mM of the crystalline cyclotetrasaccharide pentahydrate prepared in Experiment 1-1 were mixed, incubated at 37°C for two hours, and measured for the activities of sucrase and maltase. Table 10 shows the results of the reduction of the activities of sucrase and maltase in the rat small intestinal mucosal enzyme solution by radical oxidation.

### Assay for sucrase activity

To a half milliliter of a sucrose solution, which had been prepared by dissolving sucrose in 50 mM sodium maleate buffer (pH 6.6) to give a concentration of one percent (%), 0.1 ml of a AAPH-rat small intestinal mucosal enzyme solution. After incubated at 37°C for 30 min, the mixture was quantified for free glucose by the GOD method. One unit (U) activity of sucrase was defined as an enzyme amount that releases one micromole of glucose per minute from sucrose at 37° C.

### Assay for maltase activity

To a half milliliter of a maltose solution, which had been prepared by dissolving maltose in 50 mM sodium maleate (pH 6.6) to give a concentration of one percent (%), 0.05 ml of a AAPH-rat small intestinal mucosal enzyme solution. After incubated at 37°C for 30 min, the mixture was quantified for free glucose by the GOD method. One unit (U) activity of maltase was defined as an enzyme amount that releases two micromoles of glucose per minutes from maltose at 37°C.

**Table 8**

| | Formed conjugated diene (µg/mg-protein) (Conjugated diene formation: (%) ) | | |
|---|---|---|---|
| Reaction time (hr) | 0 | 2 | 6 |
| None | 0.00 (-) | 16.91 (100) | 102.98 (100) |
| Cyclotetra-saccharide | 0.00 (-) | 2.46 (15) | 61.84 (60) |
| Formation percentage (%) = (A/B) x 100 A: Formation level for conjugated diene at each time for the system with cyclotetrasaccharide B: Formation level for conjugated diene at each time for the system with no cyclotetrasaccharide | | | |

**Table 9**

| | Formed TBARS (mg/mg-protein) (TBARS formation (%)) |
|---|---|
| None | 6.10 (100) |
| Cyclotetra-saccharide | 3.71 (61) |
| Formation percentage (%) = (A/B) x 100 A: Formation level of TBARS at each time for the with cyclotetrasaccharide B: Formation level of TBARS at each time for the with no cyclotetrasaccharide | |

**Table 10**

| | Sucrase activity (u/mg-protein) (Relative residual activity (%)) | Maltase activity (u/mg-protein) (Relative residual activity (%)) |
|---|---|---|
| None | 0.0145 (-) | 0.0787 (-) |
| AAPH | 0.0042 (29) | 0.0555 (71) |
| AAPH Cyclotetrasaccharide | 0.0105 (72) | 0.0669 (85) |
| Residual activity (%) = (A/B) x 100 A: Enzyme activity of the system with AAPH B: Enzyme activity of the system with no AAPH | | |

As evident from the results in Tables 8 and 9, cyclotetrasaccharide effectively inhibited conjugated diene and TBARS formed from the oxidation of the rat intestinal mucosal enzyme solution by AAPH similarly as its inhibitory effect on the radicalization of linoleic acid in Experiment 1-4. As evident from the results in Table 10, cyclotetrasaccharide effectively inhibits the activity reduction of sucrase and maltase induced by the oxidation of rat intestinal enzyme solution by AAPH and also inhibits the modification and denaturation of the coexisting enzyme proteins induced by peroxides formed through radical reaction, as well as inhibiting the formation of free radicals and the progress of radical reaction. These results suggest that cyclotetrasaccharide inhibits intestinal disorders in rats, caused by free radicals or radical reaction.

Based on these experimental results, it was revealed that, by applying the present invention, mixtures of cyclotetrasaccharide and its saccharide derivative(s) can inhibit the formation of free radicals and the progress of radical reaction of substances which contain unsaturated compounds susceptible to cause quality deterioration and functional reduction such as smell, deterioration of color, hardening, decomposition, and denaturation whenever their decomposition is proceeded by the formation of free radicals and radical reaction during their storage. In addition, cyclotetrasaccharide and a mixture of cyclotetrasaccharide and its saccharide derivative(s) can inhibit proteins and amino acids from modification and denaturation by peroxides formed by the radicalization of unsaturated compounds. In particular, since cyclotetrasaccharide and a mixture of cyclotetrasaccharide and its saccharide derivative(s) inhibit the cytolysis including autolysis and the disorder of tissues, which are induced by radicals, it can be advantageously used as a preventive or therapeutic agent for inflammatory diseases such as burn, dermatitis, atopic dermatitis, idiopathic ulcerative colitis, gastritis, and enterocolitis, as well as a preservative for organs used in transplantation. As described above, the radical formation inhibitory agent, which contain cyclotetrasaccharide and a mixture of cyclotetrasaccharide and its saccharide derivative(s) as an effective ingredient, according to the present invention, greatly contributes to the stabilization of substances which contain unsaturated compounds, and thus it can be quite advantageously used on an industrial scale in the production, transportation, and preservation of products usable in broader fields of food products, cosmetics, pharmaceuticals, and chemical industries, which contain unsaturated compounds.

The following are the preferred embodiments of the radical reaction inhibitory agent and the compositions incorporated therewith according to the present invention, but they do not limit the scope of the present invention:

### Example 1: Radical reaction inhibitory agent

According to the method in Example 2 disclosed in Patent Literature 3, a syrup of cyclotetrasaccharide and its saccharide derivatives, having a concentration of 80%, d.s.b., and containing, d.s.b., 0.6% of glucose, 1.5% of isomaltose, 12.3% of maltose, 63.5% of cyclotetrasaccharide, 5.2% of saccharide derivatives of cyclotetrasaccharide, and 16.9% of other saccharides, was prepared from potato starch. The syrup can be preferably used as a radical reaction inhibitory agent for inhibiting the formation of free radicals and the progress of radical reaction to keep a composition stably by incorporating the agent into a composition containing an unsaturated compound such as processed foods and beverages, cosmetics, quasi drugs (or medicated cosmetics)), pharmaceuticals, feeds, pet foods including baits, and chemicals for industry, which easily deteriorates and reduces in functions such as generation of bad smell, discoloration, stiffening, decomposition, denaturation, and the like by the formation of free radicals and the progress of radical reaction during preservation.

### Example 2

### Radical reaction inhibitory agent

According to the method in Example 9 disclosed in Patent Literature 3 (except for treating with α-glucosidase and glucoamylase), a syrup of cyclotetrasaccharide and its saccharide derivatives, having a concentration of 73% (w/w), d.s.b., and containing, d.s.b., 4.1% of glucose, 8.1% of disaccharides including maltose and isomaltose, 4.0% of trisaccharides including maltotriose, 36.5% of cyclotetrasaccharide, 17.6% of saccharide derivatives of cyclotetrasaccharide, and 29.7% of other saccharides, was prepared from corn starch. The syrup can be preferably used as a radical reaction inhibitory agent for inhibiting the formation of free radicals and the progress of radical reaction to keep a composition stably by incorporating the agent into a composition containing an unsaturated compound such as processed foods and beverages, cosmetics, quasi drugs (or medicated cosmetics)), pharmaceuticals, feeds, pet foods including baits, and chemicals for industry, which easily deteriorates and reduces in functions such as generation of bad smell, discoloration, stiffening, decomposition, denaturation, and the like by the formation of free radicals and the progress of radical reaction during preservation.

### Example 3

### Radical reaction inhibitory agent

In accordance with the method in Example 4 in Patent Literature 3, a syrup containing cyclotetrasaccharide and its saccharide derivatives, prepared from tapioca starch by the method as described in Experiment 1-1, was purified, concentrated, crystallized, and dried according to the methods described in Examples 6 and 7 of Patent Literature 3, and then crystalline cyclotetrasaccharide pentahydrate with a purity of 99.6%, d.s.b., was obtained. The syrup can be preferably used as a radical reaction inhibitory agent for inhibiting the formation of free radicals and the progress of radical reaction to keep a composition stably by incorporating the agent into a composition containing an unsaturated compound such as processed foods and beverages, cosmetics, quasi drugs (or medicated cosmetics)), pharmaceuticals, feeds, pet foods including baits, and chemicals for industry, which easily deteriorates and reduces in functions such as generation of bad smell, discoloration, stiffening, decomposition, denaturation, and the like by the formation of free radicals and the progress of radical reaction during preservation.

The above crystalline cyclotetrasaccharide pentahydrate was dried according to the methods in Experiments 31 and 32 disclosed in Patent Literature 3 to produce a powdery crystalline cyclotetrasaccharide monohydrate and a powdery anhydrous crystalline cyclotetrasaccharide. The above two kinds of cyclotetrasaccharide can be advantageously used as a radical reaction inhibitory agent or a powdery crystalline cyclotetrasaccharide pentahydrate. Further, the two kinds of cyclotetrasaccharide can be advantageously used as a dryer for pulverizing volatile flavors, organic pigments, lipophilic vitamins such as vitamin A, vitamin D, vitamin E, and the like, which are easily affected by radical oxidation, while inhibiting the radical oxidation under the conditions of ambient temperature and atmospheric pressure.

### Example 4

### Radical reaction inhibitory agent

Forty parts by weight of "MABIT™", an anhydrous crystalline maltitol commercialized by Hayashibara Shoji Inc., Okayama, Japan, was admixed with 60 parts by weight of the crystalline cyclotetrasaccharide pentahydrate, obtained in Example 3, to prepare a powdery mixture. The product can be preferably used as a radical reaction inhibitory agent for inhibiting the formation of free radicals and the progress of radical reaction to keep a composition stably by incorporating the agent into a composition containing an unsaturated compound such as processed foods and beverages, cosmetics, quasi drugs (or medicated cosmetics)), pharmaceuticals, feeds, pet foods including baits, and chemicals for industry, which easily deteriorates and reduces in functions such as generation of bad smell, discoloration, stiffening, decomposition, denaturation, and the like by the formation of free radicals and the progress of radical reaction during preservation.

### Example 5

### Radical reaction inhibitory agent

Fifty parts by weight of "TREHA™", a food-grade hydrous crystalline α,α-trehalose commercialized by Hayashibara Shoji Inc. , Okayama, Japan, was admixed with 50 parts by weight of the crystalline cyclotetrasaccharide pentahydrate, obtained in Example 3, to prepare a powdery mixture. The product can be preferably used as a radical reaction inhibitory agent for inhibiting the formation of free radicals and the progress of radical reaction to keep a composition stably by incorporating the agent into a composition containing an unsaturated compound such as processed foods and beverages, cosmetics, quasi drugs (or medicated cosmetics)), pharmaceuticals, feeds, pet foods including baits, and chemicals for industry, which easily deteriorates and reduces in functions such as generation of bad smell, discoloration, stiffening, decomposition, denaturation, and the like by the formation of free radicals and the progress of radical reaction during preservation. The product can be easily used intact or in the form of a granule or a tablet which can be prepared by incorporating sugar esters and granulating or making into a tablet.

### Example 6

### Radical reaction inhibitory agent

"TREHA™", a food-grade hydrous crystalline α,α-trehalose commercialized by Hayasibara Shoji Inc., Okayama, Japan, was dissolved in water and then concentrated under a reduced pressure with heating to 60°C to prepare a trehalose solution having a concentration of 75%, d.s.b. The concentrated trehalose solution was kept under ambient temperature to crystallize, α-trehalose. The resulting crystal was washed twice with water, dried, and pulverized to prepare a powdery hydrous crystalline, α,α-trehalose with a purity of 99.8%, d.s.b. Fifty parts by weight of the powdery crystalline α,α-trehalose was admixed with 50 parts by weight of the crystalline cyclotetrasaccharide pentahydrate, obtained in Example 3, to produce a powdery mixture. The product can be preferably used as a radical reaction inhibitory agent for inhibiting the formation of free radicals and the progress of radical reaction to keep a composition stably by incorporating the agent into a composition containing an unsaturated compound such as processed foods and beverages, cosmetics, quasi drugs (or medicated cosmetics)), pharmaceuticals, feeds, pet foods including baits, and chemicals for industry, which easily deteriorates and reduces in functions such as generation of bad smell, discoloration, stiffening, decomposition, denaturation, and the like by the formation of free radicals and the progress of radical reaction during preservation. Since the product is composed of cyclotetrasaccharide with a high purity and α,α-trehalose, it has a low reactivity and is stable. Therefore, the product can be preferably used for a composition which has amino groups and easily causes the deterioration of quality and Maillard reaction with reducing sugars. The product can be easily used intact or in the form of a granule or a tablet which can be prepared by incorporating sugar esters and granulating or making into a tablet.

### Example 7

### Radical reaction inhibitory agent

Two parts by weight of L-ascorbic acid, one part by weight of vitamin E, and 0.5 part by weight of glycerin-fatty acid ester were admixed with 70 parts by weight of the syrup containing cyclotetrasaccharide and its saccharide derivatives, obtained in Example 1, to prepare a composition. The product can be preferably used as a radical reaction inhibitory agent for inhibiting the formation of free radicals and the progress of radical reaction to keep a composition stably by incorporating the agent into a composition containing an unsaturated compound such as processed foods and beverages, cosmetics, quasi drugs (or medicated cosmetics)), pharmaceuticals, feeds, pet foods including baits, and chemicals for industry, which easily deteriorates and reduces in functions such as generation of bad smell, discoloration, stiffening, decomposition, denaturation, and the like by the formation of free radicals and the progress of radical reaction during preservation.

### Example 8

### Radical reaction inhibitory agent

Two parts by weight of L-ascorbic acid 2-glucoside commercialized by Hayashibara Biochemical Laboratories Inc. , Okayama, Japan, and two parts by weight of "αG-RUTIN™", an enzyme-treated rutin commercialized by Toyo Sugar Refining Co. Ltd., Tokyo, Japan, were admixed with 70 parts by weight of the crystalline cyclotetrasaccharide pentahydrate, obtained in Example 3, to prepare a powdery mixture. The product can be preferably used as a radical reaction inhibitory agent for inhibiting the formation of free radicals and the progress of radical reaction to keep a composition stably by incorporating the agent into a composition containing an unsaturated compound such as processed foods and beverages, cosmetics, quasi drugs (or medicated cosmetics), pharmaceuticals, feeds, pet foods including baits, and chemicals for industry, which easily deteriorates and reduces in functions such as the generation of bad smell, discoloration, stiffening, decomposition, denaturation, and the like by the formation of free radicals and the progress of radical reaction during preservation.

### Example 9

### Fish paste

Two hundred parts by weight of the radical reaction inhibitory agent, prepared in Example 1, and five parts by weight of sodium citrate were admixed with 4,000 parts by weight of a raw fish meat of Alaska pollack presoaked in water, and the resulting mixture was ground and frozen at -20°C to produce a frozen ground fish meat. After preserving the ground fish meat at -20° C for 90 days, the ground fish meat was defrosted. While, 80 parts by weight of sodium glutamate, 200 parts by weight of potato starch, eight parts by weight of sodium tripolyphosphate, 120 parts by weight of sodium chloride, and 10 parts by weight of maltitol were admixed with 300 parts by weight of ice water and dissolved therein. To 100 parts by weight of the resulting solution was added the above defrosted fish meat, and the mixture was ground into a fish paste. One hundered and twenty grams of the resulting fish paste were attached unto wooden plates and steamed to give an internal temperature of about 80° C within 30 min. Successively, the cooked fish paste was cooled under atmospheric conditions and preserved at 4°C for 24 hours to make into a cooked fish paste product. Since cyclotetrasaccharide and/or the mixture of cyclotetrasaccharide and its saccharide derivatives give proteins a satisfactory tolerance to freezing, the freshness of the above frozen ground fish meat of Alaska pollack can be sufficiently kept even after preservation by freezing. The cooked fish paste product, prepared by using the ground fish meat as a material, has a satisfactory flavor, fine texture, and glossy property. Further, cyclotetrasaccharide and/or the mixture of cyclotetrasaccharide and its saccharide derivatives stabilize unsaturated compounds including fats and inhibit the modification and denaturation of proteins and amino acids by peroxide radicals of unsaturated compounds. Therefore, the cooked fish paste product has a satisfactory preservability.

### Example 10

### Agent for keeping the freshness of fresh marine products

One hundred parts by weight of the powdery mixture, which had been prepared in Example 5 by mixing equal amounts of cyclotetrasaccharide and α,α-trehalose, 10 parts by weight of crystalline citric acid, one part by weight of an enzyme-treated rutin, and two parts by weight of polyphenol were mixed to homogeneity to make into a powdery agent for keeping the freshness of fresh marine products. The radical reaction of fish lipid is inhibited by cyclotetrasaccharide and the denaturation of fish meat by freezing is inhibited by synergism of cyclotetrasaccharide, α,α-trehalose, polyphenol, organic acid, and the enzyme-treated rutin. Therefore, the product can be preferably used as an agent for keeping the freshness of fresh marine products or their frozen products.

Four hundred parts by weight of the agent was dissolved in 12 liters of sea water and then cooled by admixing with four kilograms of crashed commercial ice. Five kilograms of mackerel obtained just after uploaded at a fishing port was soaked in the above solution and collected after 10 hours. The soaked mackerel showed no difference in appearance on its surface with that just after uploaded. Further, the mackerel showed no change in surface and kept freshness even after sequentially placing into a sealed container, preserving at -20°C for 10 days in a freezer, and defrosting.

### Example 11

### Boiled rice

Three hundred parts by weight of rice, which had been washed and drained off, was admixed with water in an amount of 1.25-folds by weight of the rice and 13.5 parts by weight of the radical reaction inhibitory agent, prepared in Example 4. After soaking the rice in the solution, the rice was boiled using a rice cooker for home use to make into boiled rice. Since cyclotetrasaccharide stabilizes unsaturated compounds such as lipids contained in the boiled rice and inhibits the modification and the denaturation of proteins and amino acids by radicalized peroxides generated from unsaturated compounds, the boiled rice has a feature that the generation of bad smell from rice bran is inhibited and the preferable flavor just after cooking is kept for a relatively long period of time. Further, since cyclotetrasaccharide effectively prevents both the retrogradation of starch and the denaturation of proteins by refrigeration or freezing, the product can be advantageously used as a boiled rice for preserving under chilled or frozen conditions susceptible to cause retrogradation and denaturation by freezing.

### Example 12

### Powdery fat

After mixing 100 parts by weight of a soybean salad oil, one part by weight of lecithin, and 10 parts by weight of water at ambient temperature, and the mixture was admixed with 100 parts by weight of the radical reaction inhibitory agent, prepared in Example 5. The resulting mixture was pulverized and sieved into a powdery fat. Cyclotetrasaccharide inhibits the formation of free radicals and the progress of radical reaction of unsaturated compounds in the product. α,α-Trehalose stabilizes unsaturated compounds by forming association products with the unsaturated compounds. Therefore, in the case of incorporating the product into various food materials,the product stabilizes unsaturated compounds contained in food materials. Also, in the case of incorporating the product into food materials which contain proteins or amino acids, the product inhibits the modification and denaturation of proteins and amino acids by peroxide radicals formed from the unsaturated compounds. Therefore, the product can be advantageously used as a seasoning material for mayonnaise, dressings, and the like, or for producing intubation feedings with high calorie, premixed feeds, and the like.

### Example 13

### Salad dressing

Twenty parts by weight of a distilled white vinegar, 15 parts by weight of a vegetable oil, five parts by weight of sucrose, two parts by weight of sodium chloride, one part by weight of a garlic powder, 0.7 part by weight of an onion powder, 0.1 part by weight of a grand white pepper, 0.3 part by weight of xanthan gum, 0.1 part by weight of potassium sorbate, and 15 parts by weight of the powdery radical reaction inhibitory agent, prepared in Example 3, were admixed with 40.8 parts by weight of water to make into a salad dressing. Since cyclotetrasaccharide inhibits radical reaction, deterioration of fatty components, deterioration of flavors of the garlic powder, onion powder, grand white pepper, and others, the quality of the salad dressing can be kept stably for a relatively long period of time. Since cyclotetrasaccharide can be used as a substitute for fats and provides creamy and rich taste, the dressing keeps a preferable taste even though it has only a half amount of vegetable oils compared with ordinary dressings and it is quite low in calorie as compared with conventional ones prepared with fats. Since the dressing contains cyclotetrasaccharide in its water-phase, the water-phase more quickly and clearly separates from oil phase than conventional dressings with only sucrose as a saccharide. Thus, the product is a high quality salad dressing.

### Example 14

### Cream

Thirty-five parts by weight of a fat mixture, prepared by mixing 80 parts of a shea fractionated butter (melting point: 38°C) and 20 parts of a rapeseed oil (melting point: 35°C); 0.3 part by weight of soybean lecithin (HLB 3); 0.03 part by weight of monoglycerin-fatty acid ester; 0.15 part by weight of hexaglycerin penta-ester; 60 parts by weight of water; four parts by weight of skim milk; and 0.1 part by weight of phosphoric acid-alkaline metal salt were mixed and preliminary emulsified by conventional method. The emulsion was homogenized under a pressurized condition of 70 kg/cm². The resulting homogenate was sterilized by heating at 145° C for few seconds and homogenized under a pressurized condition of 70 kg/cm² again. The resulting homogenate was cooled and aged for about 24 hours to make into a foaming emulsion. Eight parts by weight of the powdery radical reaction inhibitory agent, prepared in Example 3, were admixed with 100 parts by weight of a foaming emulsion and whipped using "KENWOOD MIXER", a commercialized mixer, for two minutes and 45 seconds to make into a cream with an overrun of 75%. Since cyclotetrasaccharide inhibits radical reaction, the deterioration of flavor of the product was inhibited. The cream showed satisfactory shape-keeping property, flavor, and meltability in the mouth even after preserving at 5 to 20°C for seven days or defrosting after preserving at -20°C for 14 days. Also, the cream showed no crack during preservation by freezing.

### Example 15

### Edible film

Thirty parts by weight of "PI-20™", a pullulan product commercialized by Hayashibara Shoji Inc., Okayama, Japan, and 1.5 parts by weight of the radical reaction inhibitory agent, prepared in Example 2, were admixed with 70 parts by weight of water and completely dissolved. To 100 parts by weight of the resulting solution, one part by weight of carrageenan and 0.1 part by weight of lecithin were added and dissolved to make into a homogenous solution. According to conventional method, the resulting solution was poured and spread over a polyester film with a spreading speed of 3 m/min to make into a film having a thickness of 0.03 mm, and then dried with hot blow at 90°C to make into a film product. The product is an edible film which does not quickly dissolve in water but gradually dissolves and disintegrates. The product is characteristic of being stable during preservation because the stability of unsaturated compounds in the product is improved by the mixture of cyclotetrasaccharide and its saccharide derivatives. Therefore, the product can be advantageously used in the fields of food products and pharmaceuticals, as well as cachets and the like.

### Example 16

### Roasted almond

One hundred parts by weight of a selected almond were roasted by conventional method. Three parts by weight of a solution, prepared by dissolving the radical reaction inhibitory agent obtained in Example 5 into water to give a concentration of 20%, was sprayed uniformly over the hot roasted almond under stirring conditions, and successively dusted the almond with sodium chloride to obtain a roasted almond. The product is a roasted almond with preferable flavor and has satisfactory preservability. Therefore, the product is edible per se and it can be preferably used as a material for confectionaries and breads.

### Example 17

### Green-tea drink (or beverage)

To 25 grams of a green-tea leaf 800 grams of 70°C deionized water was added to extract tea constituents for six minutes, and the resulting tea leaf residues were removed by filtration to obtain 700 ml of an extract. After diluting the extract with 5-fold volumes of deionized water, L-ascorbic acid was added to the filtrate to give a concentration of 450 ppm and the pH of the resulting solution was adjusted to 6.2 using sodium bicarbonate. Successively, the powdery crystalline cyclotetrasaccharide pentahydrate, prepared in Example 3, was added to the solution in an amount of 0.2% (w/w) to the solution, and 350 ml aliquots of the resulting solution were injected into heat-resistant glass vessels. The glass vessels were sealed and sterilized by keeping at 121°C for 10 minutes to make into a green-tea drink. Since cyclotetrasaccharide has substantially no sweetness, flavors inherent to green tea are not changed. The product is a green-tea drink with a preferable flavor, which the browning is inhibited and which keeps its green color even after preserving at 20°C for six months. Further, the preservability of the product can be improved by incorporating an enzyme-treated rutin.

### Example 18

### Emulsion containing fat

Twelve grams of a purified soybean lecithin and 25 grams of glycerin were admixed with 100 grams of a purified soybean oil and heated in a water bath and then emulsified using "POLYTRON™ HOMOGENIZER", a commercialized homogenizer. The resulting emulsion was further emulsified using "µ-MICROFURUIDIZER™", a commercialized emulsifying machine. To the resulting emulsion, 250 ml of an aqueous solution dissolving 80 grams of the radical reaction inhibitory agent obtained in Example 5, and distilled water were added to give a total volume of 1,000 ml. Successively, the solution was adjusted to give a pH of 7.2 and the resulting solution was filtrated using a membrane filter. Then, the resulting filtrate was poured into a 250 ml-vial and sterilized by autoclaving at 120°C for 20 minutes to make into an emulsion containing fats and cyclotetrasaccharide. Since the radical-reaction of the product is inhibited by cyclotetrasaccharide, the formation of organic acids including formic acid as a problem in the case of using saccharides including glucose, and the formation of free fatty acids which are harmful to living bodies are well inhibited. The product is an emulsion containing fats, which has satisfactory stability over a relatively long period or time, and it can be advantageously used alone or in combination with amino acids and vitamins as a nutritional supplement administered orally or parenterally.

### Example 19

### Flavor

Five grams of sucrose-fatty acid ester (HLB 15) and 75 grams of the radical reaction inhibitory agent, prepared in Example 4, were admixed with 100 grams of water and dissolved, and the resulting solution was sterilized by heating at 85 to 90°C for 15 minutes. After cooling the solution to about 40°C, 10 grams of a lemon oil was admixed with the solution with stirring using a homogenizer to obtain an emulsion. The product can be easily processed into a powdery flavor by drying by the method such as spray-drying. The product and the pulverized lemon oil flavor keep a preferable flavor and taste without generating bad smell even when preserved for a relatively long period of time.

### Example 20

### Vitamin E preparation

Vitamin E (α-tocopherol) commercialized by Wako Pure Chemical Industries Ltd., Tokyo, Japan, was dissolved in ethanol (a special grade reagent, commercialized by Katayama Chemical Industries Co., Ltd., Osaka, Japan) to make into an ethanol solution with a vitamin E content of 10%. Ten grams of the powdery anhydrous cyclotetrasaccharide, prepared in Example 3, were placed in a glass mortar and then a small amount of ethanol or acetic acid was added to the cyclotetrasaccharide. Using a glass rod, the mixture was repeatedly mixed with additional ethanol or acetic acid until the mixture could not keep its powdery form. Thus, a vitamin E preparation was obtained. As controls, vitamin E preparations were prepared by using " ISOELITE P™", a powdery branched cyclodextrin commercialized by Ensuiko Sugar Refining Co. , Ltd. , Tokyo, Japan, a powdery anhydrous crystalline α,α,α-trehalose prepared according to the method disclosed in Japanese Patent Kokai No. 213,283/95, "PINEFIBER™", a powdery dextrin product commercialized by Matsutani Chemical Industries Co., Ltd., Hyogo, Japan, or a soluble starch commercialized by Katayama Chemical Industries Co., Ltd., Osaka, Japan, except for using cyclotetrasaccharide by the same procedure. Successively, each powdery vitamin E preparation was dried under a reduced pressure by keeping the preparation at ambient temperature for five hours in a desiccators containing (P₂O₅) to evaporate ethanol. About 0.5 gram each of the five powdery vitamin E preparations containing any one of those saccharides was weighted and placed in 20 ml-glass vials and sealed. These five samples were preserved under a normal pressure at 60°C and the amount of vitamin E remaining in the samples was examined just after their preparation, after preserving for one day, two days, seven days, and fourteen days, respectively. The amount of vitamin E in each sample was determined by: adding 10 ml of ethanol to each of the vials containing the test samples; extracting vitamin E by shaking the resulting suspensions; centrifuging the suspensions at 10,000 rpm for 10 minutes; and measuring the amount of vitamin E in the resulting supernatants by gas-chromatography (GC). GC was carried out using "GC-14A", a GL apparatus produced by Shimadzu Corporation, Kyoto, Japan, installed with "DB-1", a column (internal diameter: 0.25 mm, length: 30 m) commercialized by Agilent Technologies, USA (former name; J & W Scientific, USA). The amount of vitamin E (mg) per one gram of a vitamin E preparation and the residual vitamin E (%) was calculated and expressed with a relative value, when the amount of vitamin E in each test sample just after its preparation was regarded as 100%, as shown in Table 11.

**Table 11**

| Saccharide | Amount of vitamin E (mg) (Residual vitamin E (%)) | | | | |
|---|---|---|---|---|---|
| | Time elapsed after preservation (days) | | | | |
| | 0 | 1 | 3 | 7 | 14 |
| Cyclotetrasaccharide | 75.9 (100) | 74.4 (98) | 72.0 (95) | 68.5 (90) | 46.3 (61) |
| Branched cyclodextrin | 34.6 (100) | 32.5 (94) | 27.0 (78) | 18.5 (53) | 12.4 (36) |
| α,α-Trehalose | 36.7 (100) | 36.2 (99) | 31.0 (85) | 26.6 (73) | 15.1 (41) |
| Dextrin | 29.8 (100) | 24.9 (84) | 22.6 (76) | 18.1 (61) | 5.0 (17) |
| Soluble starch | 19.3 (100) | 16.6 (86) | 14.9 (77) | 11.1 (57) | 5.8 (30) |
| In the table, the residual vitamin E (%) is expressed with a relative value, when the amount of vitamin E in each test sample just after its preparation was regarded as 100%. | | | | | |

As is evident from the results in Table 11, in the case of a powdery vitamin E preparation prepared by using cyclotetrasaccharide as a saccharide , the residual vitamin E (%)value was higher in comparison with the cases of powdery vitamin E preparations containing branched cyclodextrin, α,α-trehalose, dextrin, or soluble starch. In the case of vitamin E preparation containing a radical reaction inhibitory agent (cyclotetrasaccharide) of the present invention, cyclotetrasaccharide inhibits the radical reaction and decomposition of vitamin E by oxidation even under severe conditions at normal pressures and at 60°C. Therefore, in the case of such a vitamin E preparation containing cyclotetrasaccharide, the process control for pulverization and preparation thereof is easy. Usually, vitamin E preparation was preserved with a deoxidizer or after enclosing in a capsule having non-permeability against oxygen. Even under such preserving conditions, the vitamin E preparation containing cyclotetrasaccharide can be advantageously used as a vitamin E preparation which is stable for a relatively long period of time in comparison with preparations containing saccharides free of cyclotetrasaccharide.

### Example 21

### Vitamin D tablet

Vitamin D₃ (cholecalciferol), as vitamin D, commercialized by Wako Pure Chemical Industries Ltd., Tokyo, Japan, was dissolved in ethanol with the highest purity, commercialized by Katayama Chemical Industries Co., Ltd., Osaka, Japan, to make into an ethanol solution with a vitamin D₃ content of 3%. To eight grams of the powdery anhydrous cyclotetrasaccharide, prepared in Example 3, was added 1.5 ml of the above ethanol solution and the resulting mixture was pulverized similarly as in Example 20, dried under a reduced pressure, and made into a powdery vitamin D₃ preparation containing cyclotetrasaccharide. As controls, powdery vitamin D₃ preparations were prepared similarly as above by using "ISOELITE P™", a branched cyclodextrin product used in Example 20, powdery anhydrous crystalline α,α-trehalose used in Example 20, "FINETOSE™", an anhydrous crystalline maltose commercialized by Hayashibara Shoji Inc., Okayama, Japan, or "FUNACELL SF™", a cellulose product commercialized by Funakoshi Co., Ltd., Tokyo, Japan, except for using cyclotetrasaccharide. About 0.3 gram each of the powdery vitamin D₃ preparations containing any one of the above saccharides was made into a vitamin D₃ tablet by pressurizing at 200 kg/cm² for two minutes using a tablet machine. Vitamin D₃ tablets thus obtained were weighted and each one tablet of which was placed in a 20 ml-glass vial and sealed to obtain five kinds of vitamin D₃ tablets containing a saccharide. The vials were preserved under a normal pressure at 60°C and the amount of vitamin D₃ remaining in the vials was examined just after preparation, after preserving for two hours, one day, two days, and seven days, respectively. The amount of vitamin D₃ in each tablet was determined by: adding 10 ml of 70% aqueous ethanol solution to each vial containing any of the test samples; crushing the tablets using a glass rod before extracting vitamin D₃; filling up the total volume to 50 ml with 70% aqueous ethanol solution; centrifuging the resulting solutions at 10,000 rpm for 10 minutes; filtrating the resulting supernatants; and measuring the absorbance at a wavelength of 265 nm for the resulting filtrates. The amount of vitamin D₃ was calculated based on the absorbance coefficient (E_{1%}) at a wavelength of 265 nm, which was regarded as 475. The amount of vitamin D₃ (mg) per one gram of each of the vitamin D₃ tablets and the residual vitamin D₃ (%) was calculated based on the amount of vitamin D₃ in the test samples just after preparation, which was regarded as 100%. The results are in Table 12.

**Table 12**

| Saccharide | Amount of vitamin D₃ (mg) (Residual D₃ percentage (%)) | | | | |
|---|---|---|---|---|---|
| | Time after preparation | | | | |
| | Just after its preparation | Two hours | One day | Two days | Seven days |
| Cyclotetrasaccharide | 5.27 (100) | 4.57 (87) | 3.49 (66) | 2.91 (55) | 2.65 (50) |
| Branched cyclodextrin | 5.36 (100) | 0.87 (16) | 0.82 (15) | 0.82 (15) | 0.87 (16) |
| α,α-Trehalose | 4.67 (100) | 1.58 (34) | 1.11 (24) | 1.02 (22) | 1.05 (22) |
| Dextrin | 4.93 (100) | 1.26 (26) | 0.93 (19) | 0.95 (19) | 0.90 (18) |
| Cellulose | 4.51 (100) | 1.46 (32) | 1.05 (23) | 1.00 (22) | 0.95 (21) |
| Residual vitamin D₃ (%) represents a relative value calculated based on the amount of vitamin D₃ in each sample just after its preparation, which was regarded as 100%. | | | | | |

As is evident from the results in Table 12, after preserving for seven days, the residual vitamin D₃ (%) values in the vitamin D₃ tablets containing a branched cyclodextrin, α,α-trehalose, dextrin, or soluble starch were in the range of 53 to 75%, but in the case of the vitamin D₃ tablet containing cyclotetrasaccharide, 90% of vitamin D₃ was remained. After preserving for 14 days, the residual vitamin D₃ (%) values of the tablets containing other saccharides was about 40% or lower, but the value of the tablet containing cyclotetrasaccharide was 61%. In the case of the vitamin D₃ tablet containing a radical reaction inhibitory agent (cyclotetrasaccharide) of the present invention, cyclotetrasaccharide inhibits radical reaction and decomposition of vitamin D₃ by oxidation even under the severe conditions of normal pressures and at 60°C, compared with vitamin D₃ tablets containing other saccharides. Therefore, the vitamin D₃ tablets containing cyclotetrasaccharide of the present invention is easily controlled its processes of pulverization and tabletting. Usually, vitamin D₃ tablets are preserved with a deoxidizer or after being coated with a film having non-permeability against oxygen. Even under such preserving conditions, such vitamin D₃ tablets containing cyclotetrasaccharide can be advantageously used as a vitamin D₃ tablet which is stable for a relatively long period of time, compared with tablets containing saccharides free of cyclotetrasaccharide.

### Example 22

### Cosmetic cream for dermatological external use

Five parts by weight of propylene glycol and 10 parts by weight of the radical reaction inhibitory agent, prepared in Example 8, were admixed with 48 parts by weight of refined water and dissolved by heating at 70°C to prepare a water-phase. While, 40 parts by weight of squalane, 15 parts by weight of reduced lanoline, 10 parts by weight of beeswax, eight parts by weight of oleyl alcohol, five parts by weight of fatty acid glyceride, three parts by weight of polyoxyethlene sorbitan monooleic acid ester (20 E. O.), three parts by weight of lipophilic glycerin monostearate, one part by weight of dipotassium glycyrrhizinate, two parts by weight of an indigo extract with water, and appropriate amounts of a flavor and an antioxidant were admixed to homogeneity at 70° C to prepare an oil-phase. The oil-phase was admixed with the water-phase and emulsified preliminary according to conventional method. Then, the resulting emulsion was further emulsified to homogeneity using a homogenizer and cooled to make into an oil-in-water cream for dermatological external use. The product exhibits a preferable moisture-keeping property to the skin. Further, the product can be advantageously used as a dermatological external agent with a high quality, having a satisfactory preservation stability because unsaturated compounds in oily components, which are mainly responsible for moisture-retaining ability, are stabilized by cyclotetrasaccharide and α,α-trehalose. Cyclotetrasaccharide.in the cream inhibits a radical reaction of fats and glycyrrhizinoic acid and/or the components from indigo inhibit the skin inflammation through their various functions such as inhibition of hyaluronidase activity. As a result, the cream can be used for inhibiting the aging of the skin as represented by speckle, wrinkle, and the like; and for keeping the skin to the desired fresh and youthful skin.

### Example 23

### Eye-drops

Four and half parts by weight of the radical reaction inhibitory agent, prepared in Example 6, 0.4 part by weight of sodium chloride, 0.15 part by weight of potassium chloride, 0.2 part by weight of sodium dihydrogen phosphate, 0.15 part by weight of borax, and 0.1 part by weight of L-ascorbic acid 2-glucoside commercialized by Hayashibara Biochemical Laboratories Inc., Okayama, Japan, were dissolved in sterilized refined water to give a total volume of 100 ml. The solution was prepared into a sterilized preparation by conventional method to make into an eye-drop . The eye-drop was adjusted to give a pH of 7.3. Cyclotetrasaccharide contained in the radical reaction inhibitory agent inhibits inflammation of eyes and α,α -trehalose exhibits the effect of preventing mucous membrane of the eye from drying. Further, L-ascorbic acid 2-glucoside continuously supplies vitamin C while being gradually hydrolyzed by an enzyme. Therefore, the eye-drop can be advantageously used for preventing and curing visual fatigue, allergic inflammation of the eye, disorders of the eye such as dry-eye, and it can be used as an eye-wash.

### Example 24

### Ointment for curing wound

To 500 parts by weight of the powdery crystalline cyclotetrasaccharide pentahydrate, prepared by the method in Example 3, 0.02 part by weight of "*KANKOSO* 101" was admixed. Further, 200 parts by weight of a 10% aqueous pullulan solution was admixed with the above mixture to make into an ointment for curing wound having an adequate spreadability and adhesiveness. Wounds such as cut, scratch, burn, athlete's foot, and the like can be cured by applying the product to the wounds directly or by spreading on gauzes to apply to affected parts. cyclotetrasaccharide inhibits the radical reaction of *"KANKOSO* 101" and stabilizes the preparation. Further, since the product inhibits inflammation caused by radical reaction of affected parts, the curing period can be shortened and wounds can be cured thoroughly. Furthermore, since cyclotetrasaccharide has no hemolytic action as reported on cyclodextrin but inhibits the action, the ointment for curing wound of the present invention can be advantageously used for curing hemorrhagic wound.

### Example 25

### Suppository containing interferon-α

"OIF™" for injection with 10 million units of an interferon-α, an interferon-α preparation commercialized by Otsuka Pharmaceutical Co., Ltd., Tokyo, Japan, was dissolved in 0.1 ml of distilled water. Crystalline cyclotetrasaccharide monohydrate was prepared by the steps of placing the powdery cyclotetrasaccharide prepared in Example 6 in a glass vessel and keeping the vessel in an oil-bath, preheated to 140°C, for 30 min. To 0.1 part by weight of the above interferon-α solution were added 0.9 part by weight of crystalline cyclotetrasaccharide monohydrate and nine parts by weight of "PHARMASOL™", an olegenous base, which has been preheated and solved at 40°C. Then, the mixture was poured into a mold and cooled to make into a suppository containing interferon-α. One gram of the product contains one million units of interferon-α activity. Since interferon-α in the product is stabilized, the suppository can be advantageously used for curing interferon-α susceptive diseases such as herpes virus infectious diseases, hepatitis, and malignant tumors.

### Example 26

### Enzyme preparation

Four milligrams of a lactate dehydrogenase (429 units/mg) derived from a microorganism, commercialized by Toyobo Co., Ltd., Osaka, Japan, were dissolved in 10 ml of deionized water, and 0.3 ml of the resulting solution and 0.3 ml of a solution, prepared by dissolving the cyclotetrasaccharide prepared in Example 4 in deionized water to give a concentration of 3.5% were gently stirred to mix in a glass vessel for lyophilization (Diameter: 10 mm, Height: 50 mm, for a part for keeping solution), and lyophilized by conventional method to obtain a powdery preparation of lactate dehydrogenase. Since cyclotetrasaccharide inhibits the denaturation of enzymes by freezing and drying during lyophilization, the enzyme activity in the product can be kept at the initial activity for a relatively long period of time under various preservation conditions such as ambient temperature, refrigeration, and freezing. Further, the enzyme activity of an aqueous solution dissolving the product can be kept for a relatively long period of time even when preserved under refrigeration at a temperature of about 4 to about -1°C or a freezing temperature.

### Example 27

### Freeze-dried complement preparation

After collecting blood from a guinea pig, a serum fraction was separated. While, one part by weight of the radical reaction inhibitory agent, prepared in Example 5, was admixed with nine parts by weight of "*Otsuka saline for injection*™", a physiological saline produce by Otsuka Pharmaceutical Co. , Ltd. , Tokyo, Japan, and dissolved completely to make into a solution. One part by weight of the resulting solution was admixed with one part by weight of the above serum fraction and dissolved to homogeneity with stirring. 0.2 ml aliquots of the resulting mixture were placed in 2 ml-ampoules and lyophilized under drying conditions of reaching a final temperature of 25° C according conventional method. Since cyclotetrasaccharide stabilizes unsaturated compounds including fats contained in the product and inhibits the modification and the denaturation of complements by peroxides formed by radicalization of unsaturated compounds in the serum. Therefore, a series of enzymatic reaction systems and other biological activities of complements are kept stably even when preserved for a relatively long period of time under refrigerating or ambient temperature. Further, since cyclotetrasaccharide prevents proteins from denaturation caused by freezing or drying, the product with cyclotetrasaccharide can be advantageously used as a reagent with a high quality for clinical diagnosis or immunological tests.

### Example 28

### Vinyl chloride resin

One hundred parts by weight of a vinyl chloride resin, 500 parts by weight of tetrahydrofuran, 0.001 part by weight of barium stearate, 0.001 part by weight of zinc stearate, and 0.6 part by weight of the powdery anhydrous crystalline cyclotetrasaccharide, prepared in Example 3, were mixed to homogeneity. Then, the mixture was heated to 180°C, kneaded for five minutes, and made into a sheet with a thickness of 0.5 mm. Since cyclotetrasaccharide inhibits radical reaction caused by ultraviolet ray, the product is a vinyl chloride resin having a satisfactory tolerance to light. It was revealed that cyclotetrasaccharide can be preferably used as a stabilizer for resins because the thermal stability of resins was improved by adding cyclotetrasaccharide.

### POSSIBILITY OF INDUSTRIAL APPLICABILITY

The present invention was made based on the finding that the decomposition of unsaturated compounds is progressed as a result of the formation of free radicals and the induction of radical reaction, and that cyclotetrasaccharide, which is a non-reducing saccharide composed of glucose units, and a mixture of such cyclotetrasaccharide and its saccharide derivative(s) inhibit the modification and the denaturation of proteins induced by peroxides of unsaturated compounds and exert a strong inhibitory action on the formation of free radicals and radical reactions. Based on this finding, the present invention is to inhibit the formation of free radicals and the progress of radical reactions and also inhibit the modification and the denaturation of proteins induced by peroxides of unsaturated compounds by incorporating a radical reaction inhibitory agent, which contains as an effective ingredient the above-identified cyclotetrasaccharide or the mixture, into a system with organic unsaturated compounds in which free radicals have or have not been formed or any radical reaction has been or has not been occurred. Also, since the above-identified cyclotetrasaccharide and the mixture are safe as a food and relatively highly stable, they can be widely applicable in a variety of fields such as food products, agriculture/forestry/fisheries, cosmetics, quasi-drugs (or medicated cosmetics), pharmaceuticals, daily goods, chemical industrial products, dyes, paints, building materials, flavors, chemicals, synthetic fibers, pigments, photosensitive dyes, and optical recording media. As described above, the present invention with such an outstandingly advantageous functions and effects is a significant invention that will greatly contribute to this art.

## Claims

1. A radical reaction inhibitory agent, comprising as an effective ingredient a cyclotetrasaccharide represented by cyclo{→6)-α-D-glucopyranosyl-(1→3)-α-D-glucopyranosyl-(1→6)-α-D -glucopyranosyl-(1→3)-α-D-glucopyranosyl-(1→} or a mixture of said cyclotetrasaccharide and its saccharide derivative(s).

2. The radical reaction inhibitory agent of claim 1, which further contains α,α-trehalose and/or maltitol.

3. The radical reaction inhibitory agent of claim 1 or 2, which further contains a radical scavenger.

4. The radical reaction inhibitory agent of claim 3, wherein said radical scavenger is one or more members selected from the group consisting of vitamins, flavonoids, polyphenols, terpenes, unsaturated fatty acids, and antioxidants.

5. The radical reaction inhibitory agent of any one of claims 1 to 4, which further contains one or more members selected from the group consisting of reducing saccharides, non-reducing saccharides, cyclodextrins, water-soluble polysaccharides, spices, acids, tastes, alcohols, inorganic salts, emulsifiers, flavors, and pigments.

6. The radical reaction inhibitory agent of any one of claims 1 to 5, which contains said cyclotetrasaccharide in an amount of at least 1 w/w %, on a dry solid basis.

7. A composition which the radical reaction is inhibited and which comprises an unsaturated compound and the radical reaction inhibitory agent of any one of claims 1 to 6.

8. The composition of claim 7, wherein said unsaturated compound is one or more members selected from the group consisting of fatty acids, simple lipids, conjugated lipids, terpenes, alcohols, glycosides, vitamins, proteins, peptides, amino acids, enzymes, hormones, cytokines, antibodies, flavors, pigments, dyes, emulsifiers, high molecules, and vinyls.

9. The composition of claim 8, wherein said vitamin is one or more members selected from the group consisting of vitamin D, vitamin E, and derivatives thereof.

10. The composition of any one of claims 7 to 9, which is a food product, cosmetic, quasi-drug (or medicated cosmetic), pharmaceutical, feed, pet food including bait, daily good, chemical industrial product, or a shaped product made from high molecules.

11. A method for inhibiting radical reaction, comprising a step of incorporating the radical reaction inhibitory agent of any one of claims 1 to 6 into a composition comprising an unsaturated compound.

12. A method for inhibiting radical reaction, comprising a step of incorporating the radical reaction inhibitory agent of any one of claims 1 to 6 into a composition comprising an unsaturated compound in order to prevent an ingredient in said composition other than said unsaturated compound from being denatured by a peroxide of an unsaturated compound, formed by radical reaction.

13. The method of claim 10 or 11, wherein said unsaturated compound is one or more members selected from the group consisting of fatty acids, simple lipids, conjugated lipids, terpenes, alcohols, glycosides, vitamins, proteins, peptides, amino acids, enzymes, hormones, cytokines, antibodies, flavors, pigments, dyes, emulsifiers, high molecules, and vinyls.

14. The method of claim 13, wherein said vitamin is one or more members selected from the group consisting of vitamin D, vitamin E, and derivatives thereof.

15. The method of any one of claims 11 to 14, which is a food product, cosmetic, quasi-drug (or medicated cosmetic), pharmaceutical, feed, pet food including bait, daily good, chemical industrial product, or a shaped product made from high molecules.
